# EUROPEAN PATENT APPLICATION

(11) **EP 3 404 039 A1**
(43) Date of publication of application: **21.11.2018**
(21) Application number: 16882148.6
(22) Date of filing: 30.12.2016
(51) Int. Cl.: C07K 16/18, G01N 33/543, G01N 33/68, C12N 15/63

(54) **RECOMBINANT PICP PROTEIN, AND METHOD FOR PREPARING ANTIBODY SPECIFICALLY BINDING THERETO**

(30) Priority: 30.12.2015 KR 20150189684
(71) Applicant: Medicinal Bioconvergence Research Center, Suwon-si, Gyeonggi-do 16229 (KR)
(72) Inventor: KIM, Sunghoon, Seoul 06269 (KR); SEO, Woo-Young, Suwon-si Gyeonggi-do 16310 (KR); KANG, Sujin, Incheon 21362 (KR); KIM, Yong-Sung, Suwon-si Gyeonggi-do 16534 (KR); KIM, Jeong-Ho, Busan 47803 (KR); BAEK, Du-San, Namyangju-si Gyeonggi-do 12118 (KR)
(74) Representative: Hoefer & Partner Patentanwälte mbB
(86) International application number: PCT/KR2016/015540
(87) International publication number: WO 2017/116196

(57) **Abstract**

The present invention relates to a recombinant PICP protein, and a method for preparing an antibody specifically binding thereto and, more specifically, to: an antibody specifically binding to PICP or a fragment thereof; an enzyme-linked immunosorbent assay (ELISA) kit and a composition for diagnosing metabolic bone diseases, both of which contain the same; a method for detecting PICP by using the same; a polynucleotide encoding the antibody or a fragment, and a recombinant expression vector comprising the same; cells transformed by the recombinant expression vector; and a method for preparing an antibody specifically binding to PICP or a fragment thereof by using the recombinant expression vector, and a method for preparing a recombinant PICP protein. PICP, which is a protein complex, can be easily prepared in a high purity and in a high yield by using the method of the present invention. In addition, according to the present invention, the antibody or a fragment thereof has very high binding affinity and binding specificity to PICP and has no cross-reactivity to a similar polypeptide, and an amino acid sequence important for antigen binding is specified, and thus an antibody can be readily and repeatedly mass produced. According to the present invention, the antibody and the method can be useful for developing a kit for detecting PICP or a diagnostic reagent using PICP.

## Description

### Technical Field

The present invention relates to a recombinant PICP protein and a method for producing an antibody specifically binding to the same and, more specifically, to an antibody or fragment thereof specifically binding to PICP, to an enzyme-linked immunosorbent assay (ELISA) kit including the same, to a composition containing the same for diagnosing a metabolic bone disease, to a method for detecting PICP by using the same, to a polynucleotide encoding the antibody or fragment thereof, to a recombinant expression vector including the same, to cells transfected with the recombinant expression vector, to a method for producing an antibody or fragment thereof specifically binding to PICP by using the recombinant expression vector, and to a method for producing a recombinant PICP protein.

### Background Art

This application claims priority from and the benefit of Korean Patent Application No. 10-2015-0189684 filed on 30 December 2015, which is hereby incorporated in its entirety by reference.

Collagen is a main structural protein of the extracellular matrix in animal connective tissues. Collagen is one of the most expressed proteins in mammals, and is estimated to account for approximately 25-35% of all expressed proteins. Collagen is a protein in the form of a fiber, and is mostly found in fibrous tissues such as tendons, ligaments and skin.

Of these, collagen type I is the most frequently expressed collagen in the human body, constitutes a collagen fiber, is expressed in the tendons, ligaments, muscle fibers, bones, dermis, tooth dentin, and the like, and is also found in scar tissue that remains in the wound regeneration process. Collagen type I has a triple helix structure with a heterotrimer in which collagen α1 chain (or α-1 type collagen) polypeptides and collagen α2 chain polypeptide are wound together at a ratio of 2:1. Collagen type I has a molecular weight of about 285 kDa. Collagen type 1, like other collagens, is produced from a precursor called procollagen. The procollagen further contains, in addition to collagen having a triple helix structure, propeptides, which are cleaved by particular protease, at the N-terminal and C-terminal. The α1 and α2 polypeptides synthesized in cells start to form a triple-helical fiber structure from the C-terminal propeptide in the endoplasmic reticulum. The procollagen completing a triple helix structure is secreted out of the cells, and the propeptides at the N-terminal and the C-terminal are removed by metalloproteinase in extracellular matrix, so that procollagen matures into collagen. The mature collagen cross-links each other, finally forming collagen fibers.

When the N-terminal and C-terminal propeptides are not normally cleaved during the mature of procollagen into collagen, the triple helix structure of collagen is abnormal, resulting in defects in collagen functions. In addition, collagen type 1 fibers play an important role in especially constituting and supporting bones, and thus the mutation in the α1 or α2 chain causes osteogenesis imperfect, such as brittle bone diseases. The procollagen type 1, C-terminal propeptide (PICP) is essential for the formation of a triple helix structure by procollagen type 1, and is an important indicator to measure collagen metabolism since it is isolated from the collagen mature procedure. Procollagen type 1 is produced and secreted from osteoblasts, chondroblasts, and the like. PICP may be used as a marker of bone growth and bone and joint diseases, such as osteoporosis and arthritis.

Therefore, the development of techniques capable of detecting PICP with high sensitivity is needed.

### Detailed Description of the Invention

### Technical Problem

The present inventors first fabricated a recombinant PICP protein as a protein complex, and developed a novel antibody by screening a scFv phage library and a yeast Fab library to select a fragment of an antibody fragment specific to the recombinant PICP protein and converting the fragment of an antibody into a usually used IgG format, and therefore, the present inventors completed the present invention.

Therefore, an aspect of the present inveniton is to provide an antibody or fragment thereof specifically binding to procollagen type I C-terminal propeptide (PICP), the antibody or fragment thereof comprising: a heavy chain variable domain (VH) comprising a heavy chain complementary determining region 1 (CDR1) containing an amino acid sequence selected from the group consisting of SEQ ID NO: 1, SEQ ID NO: 13, SEQ ID NO: 25, and SEQ ID NO: 37, a heavy chain complementary determining region 2 (CDR2) containing an amino acid sequence selected from the group consisting of SEQ ID NO: 3, SEQ ID NO: 15, SEQ ID NO: 27, and SEQ ID NO: 39, and a heavy chain complementary determining region 3 (CDR3) containing an amino acid sequence selected from the group consisting of SEQ ID NO: 5, SEQ ID NO: 17, SEQ ID NO: 29, and SEQ ID NO: 41; and a light chain variable domain (VH) comprising a light chain complementary determining region 1 (CDR1) containing an amino acid sequence selected from the group consisting of SEQ ID NO: 7, SEQ ID NO: 19, SEQ ID NO: 31, and SEQ ID NO: 43, a light chain complementary determining region 2 (CDR2) containing an amino acid sequence selected from the group consisting of SEQ ID NO: 9, SEQ ID NO: 21, SEQ ID NO: 33, and SEQ ID NO: 45, and a light chain complementary determining region 3 (CDR3) containing an amino acid sequence selected from the group consisting of SEQ ID NO: 11, SEQ ID NO: 23, SEQ ID NO: 35, and SEQ ID NO: 47.

Another aspect of the present inveniton is to provide an enzyme-linked immunosorbent assay (ELISA) kit comprising the antibody or fragment thereof of the present invention.

Still another aspect of the present inveniton is to provide a composition for diagnosing a metabolic bone disease, the composition comprising the antibody or fragment thereof of the present invention.

Furthermore, an aspect of the present inveniton is to provide a composition for diagnosing a metabolic bone disease, the composition consisting of the antibody or fragment thereof of the present invention.

Furthermore, an aspect of the present inveniton is to provide a composition for diagnosing a metabolic bone disease, the composition consisting essentially of the antibody or fragment thereof of the present invention.

Still another aspect of the present inveniton is to provide a use of the antibody or fragment thereof of the present invention for preparing an agent for diagnosis of a metabolic bone disease.

Still another aspect of the present inveniton is to provide a method for diagnosing a metabolic bone disease in a subject, the method comprising:
(a) obtaining a biological sample from a subject;
(b) determining the level of PICP protein in the biological sample using the antibody or fragment of the present invention; and
(c) comparing the determined level of PICP protein with the level of PICP protein in a normal subject

Still another aspect of the present inveniton is to provide a PICP-specific detection method comprising:
(1) preparing a sample;
(2) contacting the antibody or fragment of the present inveniton with the sample; and
(3) detecting the antibody or fragment thereof

Still another aspect of the present inveniton is to provide a polynucleotide encoding the antibody or fragment thereof of the present invention.

Still another aspect of the present inveniton is to provide a recombinant expression vector comprising the polynucleotide.

Still another aspect of the present inveniton is to provide a cell transfected with the recombinant expression vector.

Still another aspect of the present inveniton is to provide a method for preparing an antibody or fragment thereof specifically binding to PICP, the method comprising:
(a) transfecting host cells with the recombinant expression vector;
(b) culturing the transfected host cells to prepare an antibody or fragment thereof; and
(c) harvesting the antibody or fragment thereof prepared in the host cells.

Still another aspect of the present inveniton is to provide a method for preparing a recombinant PICP protein, the method comprising:
(i) constructing recombinant expression vectors comprising polynucleotides encoding PICP α1 chain and PICP α2 chain, respectively, to which a signal peptide stimulatin extracellular secretion and a label protein are conjugated to each of the chains;
(ii) co-transfecting cells with a mixture of the recombinant expression vector comprising the polynucleotide encoding PICP α1 chain and the recombinant expression vector comprising the polynucleotide encoding PICP α2 chain;
(iii) culturing the co-transfected cells; and
(iv) obtaining PICP protein produced in the cells.

### Technical Solution

In accordance with an aspect of the present invention, there is provided an antibody or fragment thereof specifically binding to procollagen type I C-terminal propeptide (PICP), the antibody or fragment thereof comprising: a heavy chain variable domain (VH) comprising a heavy chain complementary determining region 1 (CDR1) containing an amino acid sequence selected from the group consisting of SEQ ID NO: 1, SEQ ID NO: 13, SEQ ID NO: 25, and SEQ ID NO: 37, a heavy chain complementary determining region 2 (CDR2) containing an amino acid sequence selected from the group consisting of SEQ ID NO: 3, SEQ ID NO: 15, SEQ ID NO: 27, and SEQ ID NO: 39, and a heavy chain complementary determining region 3 (CDR3) containing an amino acid sequence selected from the group consisting of SEQ ID NO: 5, SEQ ID NO: 17, SEQ ID NO: 29, and SEQ ID NO: 41; and a light chain variable domain (VH) comprising a light chain complementary determining region 1 (CDR1) containing an amino acid sequence selected from the group consisting of SEQ ID NO: 7, SEQ ID NO: 19, SEQ ID NO: 31, and SEQ ID NO: 43, a light chain complementary determining region 2 (CDR2) containing an amino acid sequence selected from the group consisting of SEQ ID NO: 9, SEQ ID NO: 21, SEQ ID NO: 33, and SEQ ID NO: 45, and a light chain complementary determining region 3 (CDR3) containing an amino acid sequence selected from the group consisting of SEQ ID NO: 11, SEQ ID NO: 23, SEQ ID NO: 35, and SEQ ID NO: 47.

In accordance with another aspect of the present invention, there is provided an enzyme-linked immunosorbent assay (ELISA) kit comprising the antibody or fragment thereof of the present invention.

In accordance with still another aspect of the present inveniton, there is provided a composition for diagnosing a metabolic bone disease, the composition comprising the antibody or fragment thereof of the present invention.

Furthermore, in accordance with an aspect of the present inveniton, there is provided a composition for diagnosing a metabolic bone disease, the composition consisting of the antibody or fragment thereof of the present invention.

Furthermore, in accordance with an aspect of the present inveniton, there is provided a composition for diagnosing a metabolic bone disease, the composition consisting essentially of the antibody or fragment thereof of the present invention.

In accordance with still another aspect of the present inveniton, there is provided a use of the antibody or fragment thereof of the present invention for preparing an agent for diagnosis of a metabolic bone disease.

In accordance with still another aspect of the present inveniton, there is provided a method for diagnosing a metabolic bone disease in a subject, the method comprising:
(a) obtaining a biological sample from a subject;
(b) determining the level of PICP protein in the biological sample using the antibody or fragment of the present invention; and
(c) comparing the determined level of PICP protein with the level of PICP protein in a normal subject

In accordance with still another aspect of the present inveniton, there is provided a PICP-specific detection method comprising:
(1) preparing a sample;
(2) contacting the antibody or fragment of the present inveniton with the sample; and
(3) detecting the antibody or fragment thereof

In accordance with still another aspect of the present inveniton, there is provided a polynucleotide encoding the antibody or fragment thereof of the present invention.

In accordance with still another aspect of the present inveniton, there is provided a recombinant expression vector comprising the polynucleotide.

In accordance with still another aspect of the present inveniton, there is provided a cell transfected with the recombinant expression vector.

In accordance with still another aspect of the present inveniton, there is provided a method for preparing an antibody or fragment thereof specifically binding to PICP, the method comprising:
(a) transfecting host cells with the recombinant expression vector;
(b) culturing the transfected host cells to prepare an antibody or fragment thereof; and
(c) harvesting the antibody or fragment thereof prepared in the host cells.

In accordance with still another aspect of the present inveniton, there is provided a method for preparing a recombinant PICP protein, the method comprising:
(i) constructing recombinant expression vectors comprising polynucleotides encoding PICP α1 chain and PICP α2 chain, respectively, to which a signal peptide stimulating extracellular secretion and a label protein are conjugated;
(ii) co-transfecting cells with a mixture of the recombinant expression vector comprising the polynucleotide encoding PICP α1 chain and the recombinant expression vector comprising the polynucleotide encoding PICP α2 chain;
(iii) culturing the co-transfected cells; and
(iv) obtaining PICP protein produced in the cells.

Hereinafter, the present invention will be described in detail.

The present inventors successfully constructed a recombinant PICP protein as a protein triple complex, and screened a human-derived scFv phage library and a yeast Fab library to select a fragment of an antibody specifically binding to the recombinant PICP protein. It was confirmed that the selected fragment of an antibody maintained binding affinity and specificity to PICP even after conversion into an IgG type antibody, and showed little cross-reactivity to similar antigens. Therefore, the present specification provides: an antibody including heavy and light chain complementary determining regions (CDRs), which are confirmed to be specific to PICP and have low cross-reactivity to PICP, and amino acid sequences of heavy chain variable domain (VH) and light chain variable domain (VL); a method for producing an antibody; and a detection method using an antibody.

As used herein, the term "procollagen type I C-terminal propeptide (PICP)" refers to a C-terminal fragment of procollagen type 1, which is produced from the cleavage by bone morphogenic protein 1 (BMP1) as specific endopeptidase. PICP is a trimeric globular protein with two procollagen type 1 α1 chain polypeptides and one procollagen type 1 α2 chain polypeptide. The procollagen α1 chain is a protein which is composed of 1464 amino acids and encoded by COL1A1 gene in human. The procollagen α2 chain is a protein which is composed of 1366 amino acids and encoded by COL1A2 gene in human.

In the present invention, the term "antibody" is also called "immunoglobulin (Ig)" and is a generic term for proteins that are involved in biological immunity by selectively acting on antigens. A whole antibody found in nature is usually made up of two pairs of light chain (LC) and heavy chain (HC), each of which is a polypeptide composed of several domains, or two pairs of HC/LC as a basic unit. There are five types of heavy chains constituting mammalian antibodies, which are denoted by the Greek letters: α, δ, ε, γ, and µ, and different types of heavy chains constitute different types of antibodies: IgA, IgD, IgE, IgG and IgM T, respectively. There are two types of light chains constituting mammalian antibodies, which are denoted by λ and κ.

The heavy and light chains of antibodies are structurally divided into a variable region and a constant region depending on the variability of the amino acid sequence. The constant region of the heavy chain is composed of three or four heavy chain constant domains, such as CH1, CH2, and CH3 (IgA, IgD, and IgG antibodies) and CH4 domain (IgE and IgM antibodies), depending on the type of antibody, and the light chain is composed of one constant domain CL. The variable region of the heavy chain or light chain is composed of a heavy chain variable domain (VH) or a light chain variable domain (VL), respectively. The light chain and the heavy chain are linked by one covalent disulfide bond while a variable region and a constant region thereof are arranged in parallel, and two heavy chain molecules, which are linked with the light chains, are linked to each other through two covalent disulfide bonds, thereby forming a whole antibody. The whole antibody specifically binds to an antigen through the variable regions of the heavy and light chains. The whole antibody is composed of two pairs of heavy and light chains (HC-LC), and thus one whole antibody molecule has a divalent mono-specificity wherein one whole antibody molecule binds to the two same antigens through two variable regions.

The variable region containing an antigen binding site of the antibody is divided into framework regions (FR) with low sequence variability and complementary determining regions (CDRs), which are hypervariable regions with high sequence variability. In VH and VL, three CDRs and four FRs are arranged in the order of FR1-CDR1-FR2-CDR2-FR3-CDR3-FR4 in a direction from the N-terminal to the C-terminal. CDRs with the highest sequence variability in the variable region of the antibody are sites directly binding to an antigen, and are very important in the antigen specificity of the antibody.

The antibody specifically binding to PICP according to the present inveniton includes: a heavy chain variable domain (VH) including a heavy chain complementary determining region 1 (CDR1) containing an amino acid sequence selected from SEQ ID NO: 1, SEQ ID NO: 13, SEQ ID NO: 25, and SEQ ID NO: 37, a heavy chain complementary determining region 2 (CDR2) containing an amino acid sequence selected from SEQ ID NO: 3, SEQ ID NO: 15, SEQ ID NO: 27, and SEQ ID NO: 39, and a heavy chain complementary determining region 3 (CDR3) containing an amino acid sequence selected from SEQ ID NO: 5, SEQ ID NO: 17, SEQ ID NO: 29, and SEQ ID NO: 41; and a light chain variable domain (VH) including a light chain complementary determining region 1 (CDR1) containing an amino acid sequence selected from SEQ ID NO: 7, SEQ ID NO: 19, SEQ ID NO: 31, and SEQ ID NO: 43, a light chain complementary determining region 2 (CDR2) containing an amino acid sequence selected from SEQ ID NO: 9, SEQ ID NO: 21, SEQ ID NO: 33, and SEQ ID NO: 45, and a light chain complementary determining region 3 (CDR3) containing an amino acid sequence selected from SEQ ID NO: 11, SEQ ID NO: 23, SEQ ID NO: 35, and SEQ ID NO: 47.

The antibody specifically binding to PICP according to the present invention is an antibody having the following CDR configuration in the heavy chain and light chain variable regions:
a heavy chain variable domain including heavy chain complementary determining region 1 containing the amino acid sequence represented by SEQ ID NO: 1, heavy chain complementary determining region 2 containing the amino acid sequence represented by SEQ ID NO: 3, and heavy chain complementary determining region 3 containing the amino acid sequence represented by SEQ ID NO: 5; and a light chain variable domain including light chain complementary determining region 1 containing the amino acid sequence represented by SEQ ID NO: 7, light chain complementary determining region 2 containing the amino acid sequence represented by SEQ ID NO: 9, and light chain complementary determining region 3 containing the amino acid sequence represented by SEQ ID NO: 11; or
a heavy chain variable domain including heavy chain complementary determining region 1 containing the amino acid sequence represented by SEQ ID NO: 13, heavy chain complementary determining region 2 containing the amino acid sequence represented by SEQ ID NO: 15, and heavy chain complementary determining region 3 containing the amino acid sequence represented by SEQ ID NO: 17; and a light chain variable domain including light chain complementary determining region 1 containing the amino acid sequence represented by SEQ ID NO: 19, light chain complementary determining region 2 containing the amino acid sequence represented by SEQ ID NO: 21, and light chain complementary determining region 3 containing the amino acid sequence represented by SEQ ID NO: 23; or
a heavy chain variable domain including heavy chain complementary determining region 1 containing the amino acid sequence represented by SEQ ID NO: 25, heavy chain complementary determining region 2 containing the amino acid sequence represented by SEQ ID NO: 27, and heavy chain complementary determining region 3 containing the amino acid sequence represented by SEQ ID NO: 29; and a light chain variable domain including light chain complementary determining region 1 containing the amino acid sequence represented by SEQ ID NO: 31, light chain complementary determining region 2 containing the amino acid sequence represented by SEQ ID NO: 33, and light chain complementary determining region 3 containing the amino acid sequence represented by SEQ ID NO: 35; or
a heavy chain variable domain including heavy chain complementary determining region 1 containing the amino acid sequence represented by SEQ ID NO: 37, heavy chain complementary determining region 2 containing the amino acid sequence represented by SEQ ID NO: 39, and heavy chain complementary determining region 3 containing the amino acid sequence represented by SEQ ID NO: 41; and a light chain variable domain including light chain complementary determining region 1 containing the amino acid sequence represented by SEQ ID NO: 43, light chain complementary determining region 2 containing the amino acid sequence represented by SEQ ID NO: 45, and light chain complementary determining region 3 containing the amino acid sequence represented by SEQ ID NO: 47.

In addition, the antibody having a configuration of CDRs specifally binding to PICP may be an antibody characterized by including: a heavy chain variable domain containing an amino acid sequence selected from the group consisting of SEQ ID NO: 49, SEQ ID NO: 53, SEQ ID NO: 57, and SEQ ID NO: 61; and a light chain variable domain containing an amino acid sequence selected from the group consisting of SEQ ID NO: 51, SEQ ID NO: 55, SEQ ID NO: 59, and SEQ ID NO: 63.

The antibody including the heavy chain variable doamin (VH) and the light chain variable domain (VL) may be an antibody charcterized by including: a heavy chain containing an amino acid sequence of SEQ ID NO: 69, SEQ ID NO: 73, SEQ ID NO: 77, and SEQ ID NO: 81; and a light chain containing an amino acid sequence of SEQ ID NO: 71, SEQ ID NO: 75, SEQ ID NO: 79, and SEQ ID NO: 83.

Most preferaly, the antibody including the heavy chain variable doamin (VH) and the light chain variable domain (VL) may be an antibody including: a heavy chain containing the amino acid sequence of SEQ ID NO: 69 and a light chain containing the amino acid sequence of SEQ ID NO: 71; a heavy chain containing the amino acid sequence of SEQ ID NO: 73 and a light chain containing the amino acid sequence of SEQ ID NO: 75; a heavy chain containing the amino acid sequence of SEQ ID NO: 77 and a light chain containing the amino acid sequence of SEQ ID NO: 79; or a heavy chain containing the amino acid sequence of SEQ ID NO: 81 and a light chain containing the amino acid sequence of SEQ ID NO: 83.

The antibody specifically binding to PICP according to the present invention is not limited in the type thereof as long as the antibody has the foregoing combination of CDRs, or the foregoing configuration of VH and VL or heavy and light chains. Specifically, the antibody may be selected from the group consisting of IgG, IgA, IgM, IgE, and IgD, and IgG antibody is especially preferable. Alternatively, the antibody may be a monoclonal antibody derived from one B cells, and may be a polyclonal antibody derived from a plurality of B cells. However, the antibody is preferably a monoclonal antibody, which is a group of antibodies in which same amino acid sequences of heavy and light chains of the antibody are substantially the same. In addition, the antibody or fragment thereof of the present invention may be ones conjugated to an enzyme, a fluorescent material, a radioactive material, and a protein, but is not limited thereto.

The antibody of the present invention may be derived from any animal including mammals and birds, and may be preferably derived from humans. However, the antibody of the present invention may be a chimeric antibody including a portion of the antibody derived from an animal, which is different from a portion of the antibody derived from a human.

In addition, the fragment of the antibody in the present invention means a fragment of an antibody, which maintains the antigen-specific binding affinity of a whole antibody, and may be specifically in the form of Fab, F(ab)2, Fab', F(ab')2, Fv, diabody, scFv, or the like.

Fab (fragment, antigen-binding) is an antigen-binding fragment of an antibody, and is composed of one variable domain and one constant domain of each of the heavy and light chains. F(ab')₂ is a fragment produced by pepsin hydrolysis of an antibody, and F(ab')₂ has a form in which two Fab fragments are linked via disulfide bonds at the heavy-chain hinge region. F(ab') is a monomeric antibody fragment in which heavy-chain hinges are added to Fab isolated by the reduction of disulfide bonds of F(ab')₂ fragment. Fv (variable fragment) is an antibody fragment composed of only respective variable regions of the heavy and light chains. The scFv (single chain variable fragment) is a recombinant antibody fragment in which a heavy chain variable region (VH) and a light chain variable region (VL) are linked to each other via a flexible peptide linker. The diabody refers to a fragment in which VH and VL of scFv are linked by a very short linker and thus cannot be bound to each other, and bind to VL and VH of another scFv in the same form, respectively, to form a dimer.

For the purposes of the present invention, the fragment of the antibody is not limited in the structure or form thereof as long as the fragment of the antibody retains binding specificity to PICP, but may be preferably scFv. That is, the scFv according to the present invention has a configuration of CDRs or VH and VL specific to PICP, and may preferably contain an amino acid sequence of SEQ ID NO: 65 or SEQ ID NO: 67.

In addition, the fragment of the antibody according to the present may be Fab. Fab includes VH containing the amino acid sequence represented by SEQ ID NO: 57 and VL containing the amino acid sequence represented by SEQ ID NO: 59, or preferably includes VH containing the amino acid sequence represented by SEQ ID NO: 61 and VL containing the amino acid sequence represented by SEQ ID NO: 63.

The present invention provides an enzyme-linked immunosorbent assay (ELISA) kit including the antibody or fragment thereof of the present invention.

In an example of the present invention, ELISA reaction was conducted using an antibody and a fragment thereof selected using the recombinant PICP according to the present invention. Especially, in sandwich ELISA reaction using IgG containing a variable region of 2D scFv clone, the limit of detection was 1 ng/ml, and thus PICP could be precisely detected, and no cross-reactivity was shown for a pseudo protein PIIICP, indicating high utility as an ELISA kit. Therefore, an enzyme-linked immunosorbent assay (ELISA) kit is provided that can quantitatively detect PICP using the antibody or fragment thereof specifically binding to PICP according to the present invention.

The EISA kit is used to detect or quantitatively analyze a target material using an antibody binding to an enzyme, and the EISA kit is usually fabricated in the form of sandwich EILSA to reduce non-specific reactions and increase antigen sensitivity and binding specificity. Sandwich ELISA uses two types of antibodies specifically binding to a target material in order to detect the target material. A primary antibody (capture body) is attached to a surface of a reaction container, and a secondary antibody (detection antibody) linked to an enzyme for a color development reaction is contained in a reaction solution. First, a sample is added to the reaction container into which the primary antibody is immobilized, and incubation is conducted such that the target material is bound with the primary antibody, followed by again incubation with the secondary antibody, thereby forming a sandwich type complex of antibody (capture)-target material-antibody (detection). Last, a substrate for the enzyme linked to the secondary antibody is added to the reaction container to conduct a color development reaction of the enzyme, and the level of color development is measured to investigate the presence or absence and concentration of the target material contained in the sample. As the concentration of the target material in the sample becomes higher, more complexes of antibody (capture)-target material-antibody (detection) are formed, and more secondary antibodies capable of conducting the color development reaction are present in the reaction container. Therefore, the degree of color development and the concentration of the target material have positive correlation. In order to investigate the concentration of the target material, ELISA reaction is conducted using various concentrations of the target material to create a standard curve of color development according to the concentration, and the concentration of the standard material in the sample is deduced by matching the level of color development, measured by the ELISA reaction of the sample, to the standard curve.

The ELISA kit according to the present invention may be manufactured in the form of sandwich ELISA as described above, and may be composed of: a reaction container having a surface onto which the antibody or fragment thereof specifically binding to PICP by including heavy and light chain variable regions according to the present invention is immobilized; an antibody to which an enzyme for color development reaction is linked, a substrate of the enzyme for color development reaction, a reaction solution (or buffer) for color development reaction, a cofactor, and PICP protein with a series of particular concentrations as a standard material for creating a standard curve of PICP. Especially, the recombinant PICP protein prepared by the method of the present invention may be used as a standard material. The enzyme for color development reaction can be used without limitation as long as the enzyme is usually used for ELISA reaction in the art and does not inhibit the binding with PICP, and for example, horse radish peroxidase (HRP) as an enzyme and tetramethyl benzimidine (TMB) as a substrate for color development thereof may be used. TMB is oxidized by a catalytic action of HRP and developed into blue, and the level of color development is monitored by measurement of absorbance at a wavelength of 605 nm. Meanwhile, the addition of sulfuric acid (H₂SO₄) in the HRP enzyme reaction stops an enzymatic reaction, so the color development reaction does not proceed any further and the reaction solution is changed from blue into yellow. The yellow reaction solution stabilized by sulfuric acid is investigated by measurement of absorbance at a wavelength of 450 nm.

The present inveniton provides a composition for diagnosing a metabolic bone disease, the composition contianig the antibody or fragment thereof according to the present invention.

Furthermore, in accordance with an aspect of the present invention, the preent ivention provides a composition for diagnosing a metabolic bone disease, the composition consistig of the antibody or fragment thereof of the present invention.

Furthermore, in accordance with an aspect of the present invention, the preent ivention provides a composition for diagnosing a metabolic bone disease, the composition essentially consistig of the antibody or fragment thereof of the present invention.

In accordance with still another aspect of the present invention, the present inveniton provides a use of the antibody or fragment thereof of the present invention for preparing a preparation for diagnosis of a metabolic bone disease.

Type 1 collagen is the most important organic component that constitutes bones, and PICP, which is produced from the cleavage during the formation of type 1 collagen and released into the circulation system of blood, may be used as a quantitative marker of collagen synthesis closely associated with bone formation and growth. It has been reported that the level of PICP in body fluids is increased in disease, such as especially osteoporosis highly associated with bone turnover or regeneration, osteoporosis caused by hyperthyroidism or hyperparathyroidism, osteopenia prior to osteoporosis, osteogenesis imperfecta, bone cancer, and metastatic bone disease caused by breast cancer, lung cancer, prostate cancer, or the like. Therefore, the composition containing an antibody or fragment thereof specifically binding to PICP according to the present invention may be used to diagnose a metabolic bone disease associated with bone turnover or regeneration.

The metabolic bone disease in the present invention may be, specifically, osteoporosis, Paget's disease, osteodystrophy, osteogenesis imperfecta, bone cancer, metastatic bone disease, osteomalacia, osteopenia, bone atrophy, fibrous dysplasia, hypercalcemia, osteolysis, osteoarthritis, periodontal disease, or rheumatoid arthritis, and may be preferably osteoporosis, osteopenia, osteodystrophy, osteogenesis imperfecta, or metastatic bone disease.

The term "comprising" is used synonymously with "containing", "including", or "being characterized", and does not exclude additional ingredients or steps that are not mentioned in the composition and the method. The term "consisting of" excludes additional elements, steps, or ingredients that are not otherwise indicated. The term "essentially consisting of" means that in view of compositions or methods, the term includes described materials or steps as well as any material or step that does not substantially affect basic characteristics thereof.

Furthermore, the present invention provides a PICP-specific detection method including:
(1) preparing a sample;
(2) contacting the antibody or fragment of the present inveniton with the sample; and
(3) detecting the antibody or fragment thereof

In step (1), a sample for measuring the presence or absence and concentration of PICP protein using the antibody or fragment thereof according to the present invention is prepared.

A person skilled in the art can properly select a known method of detecting a protein by using an antibody, and can prepare a sample suitable for the selected method. In addition, the sample may be cells, tissues, blood, whole blood, serum, plasma, saliva, cerebrospinal fluid, or the like, which are obtained from a biopsy collected from a subject to be diagnosed for the presence or absence of a metabolic bone disease. The protein detecting method using the antibody includes, but is not limited thereto, for example, western blot, immune blot, dot blot, immunohistochemistry, enzyme-linked immunosorbent assay (ELISA), radioimmunoassay, competitive binding assay, immunoprecipitation, and the like, For example, for western blot, a preparation step may be performed, such as adding a buffer suitable for electrophoresis to a sample or cell lysate containing PICP, followed by boiling, and for immunohistochemistry, a treatment step may be performed, such as immobilizing and blocking cells or tissue slices.

In step (2), the antibody or fragment thereof according to the present is contacted with the sample prepared in step (1).

The antibody according to the present invention is an antibody or fragment thereof specifically binding to PICP and having a configuration of the foregoing CDRs, VH and VL, or heavy and light chains. The antibody may be preferably IgG, and the fragment of the antibody may be scFv or Fab. The type and range of the antibody or fragment thereof are described as above.

In step (3), the presence or absence and level of PICP protein in the sample are determined by detecting the antibody or fragment thereof according to the present invention contacted with the sample in step (2).

The antibody or fragment thereof according to the present invention for executing step (3) may be produced in a form that is directly labeled with fluorescence, a radioactive isotope, an enzyme, or the like, according to a method known in the art and thus can be detected without a separate secondary antibody. For example, radioactivity may be determined by scintillation counting, and fluorescence may be detected and quantified using a fluorescence microscope. In addition, the enzyme includes, for example, luciferase, peroxidase, galactosidase, and the like. Alternatively, the antibody or fragment thereof according to the present invention may be indirectly detected using a secondary antibody labeled with fluorescence, a radioactive isotope, an enzyme, or the like. Additionally, the antibody or fragment thereof according to the present invention is produced in a form that is conjugated to biotin and thus can be detected by incubation with appropriately labeled streptavidin.

In order to attain still another purpose of the present inveniton, the present invention provides a method for diagnosing a metabolic bone disease in a subject, the method including:
(a) obtaining a biological sample from a subject;
(b) determining the level of PICP protein in the biological sample using the antibody or fragment of the present invention; and
(c) comparing the determined level of PICP protein with the level of PICP protein in a normal subject

As used herein, the term "biological sample" or "sample" includes blood and other liquid samples originated from biology, biopsy samples, solid tissue samples such as tissue culture, or cells derived therefrom. More specifically, examples of the biological sample or sample may include, but are not limited to, tissue, extract, cell lysate, whole blood, plasma, serum, saliva, ocular fluid, cerebrospinal fluid, sweat, urine, milk, ascites fluid, synovial fluid, peritoneal fluid, and the like. The sample may be obtained from an animal, preferably a mammal, and most preferably a human being. The sample may be pre-treated before use for detection. Examples of the pretreatment may include filtration, distillation, extraction, concentration, interference ingredient deactivation, reagent addition, and the like. In addition, nucleic acids and proteins isolated from the sample may be used for detection.

According to the diagnosing method of the present invention, the actual occurrence of a disease can be diagnosed by comparing the PICP protein level in a biological sample of a normal subject with the protein level in a biological sample of a subject with a suspected metabolic bone disease. That is, the PICP protein level from a biological sample of an subject with a suspected metabolic bone disease is determined using the antibody or fragment thereof of the present invention, and the protein level from a biological sample of a normal subject is determined using the antibody or fragment thereof of the present invention, and both of the levels are compared, and then the suspected subject can be diagnosed to have a corresponding disease if the PICP protein level of the suspected subject is higher than that of the normal subject. Therefore, the diagnosing method may further include "step (d) of determining the suspected subject to have a metabolic bone disease if the PICP protein level of the suspected subject is higher than the PICP protein level of the normal subject". This is on the basis of the fact that the level of PICP in body fluids has been reported to be increased in a disease, such as especially osteoporosis highly associated with bone turnover or regeneration, osteoporosis caused by hyperthyroidism or hyperparathyroidism, osteopenia prior to osteoporosis, osteogenesis imperfect, bone cancer, and metastatic bone disease caused by breast cancer, lung cancer, prostate cancer, or the like. The metabolic bone disease is described as above.

The diagnosing method is attained through specific antigen-antibody responses of the PICP protein and the antibody or fragment thereof of the present invention, and the yield of antigen-antibody complex produced can be quantitatively determined through the size of a signal of the detection label. As used herein, the term "antigen-antibody complex" refers to a complex of PICP protein as a metabolic bone disease marker and the antibody or fragment thereof of the present invention specific thereto.

Examples of the detection label (marker) may be selected from the group consisting of an enzyme, a fluorescent material, a ligand, a luminescent material, microparticles, a redox molecule, and a radioisotope, but are not limited thereto. When the enzyme is used as the detection label, examples of the usable enzyme includes, but are not limited to, β-glucuronidase, β-D-glucosidase, β-D-galactosidase, urease, peroxidase or alkaline phosphatase, acetylcholine esterase, glucose oxidase, hexokinase and GDPase, RNase, glucose oxidase and luciferase, phospho-fructokinase, phosphoenolpyruvate carboxylase, aspartate amino transferase, phosphoenol pyruvate decarboxylase, β-latamase, and the like. Examples of the fluorescent material may include, but are not limited to, fluorescein, isothiocyanate, rhodamine, phycoerythrin, phycocyanin, allophycocyanin, o-phthaldehyde, fluorescamin, and the like. Examples of the ligand include, but are not limited to, a biotin derivative and the like. Examples of the luminescent material include, but are not limited to, acridinium ester, luciferin, luciferase, and the like. Examples of the microparticles include colloidal gold, colored latex, and the like, but are not limited thereto. Examples of the redox molecule may include, but are not limited to, ferrocene, ruthenium complexes, viologen, quinone, Ti ions, Cs ions, diimide, 1,4-benzoquinone, hydroquinone, K₄W(CN)₈, [Os (bpy)₃]²⁺, [RU (bpy)₃]²⁺, [MO(CN)₈]⁴⁻, and the like. Examples of the radioisotope may include, but are not limited to, ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, ¹⁸⁶Re, and the like.

Examples of the analysis method for determining the protein level may include, but are limited to, Western blotting, ELISA, radioimmunoassay, radioimmunodiffusion, Ouchterlony immunodiffusion, rocket immunoelectrophoresis, immunohistostaining, immunoprecipitation assay, complement fixation assay, FACS, and protein chip. Through the analysis methods, the amount of the antigen-antibody complex formed in a normal control group can be compared with the amount of the antigen-antibody complex formed in a suspected patient of metabolic bone disease, and therefore, the increase or not in the PICP yield can be determined, so that it can be diagnosed whether a disease suspected patient actually has a metabolic bone disease.

The present invention provides a polynucleotide encoding the antibody or fragment thereof according to the present invention.

A polynucleotide encoding the antibody refers to a nucleotide sequence encoding the antibody specifically binding to PICP according to the present invention, the antibody having a configuration of CDRs or a configuration of VH and VL or heavy and light chains. The foregoing polynucleotide encoding CDRs according to the present invention is described in SEQ ID NO: 2 (heavy chain CDR1), SEQ ID NO: 4 (heavy chain CDR2), SEQ ID NO: 6 (heavy chain CDR3), SEQ ID NO: 8 (light chain CDR1), SEQ ID NO: 10 (light chain CDR2), SEQ ID NO: 12 (light chain CDR3), SEQ ID NO: 14 (heavy chain CDR1), SEQ ID NO: 16 (heavy chain CDR2), SEQ ID NO: 18 (heavy chain CDR3), SEQ ID NO: 20 (light chain CDR1), SEQ ID NO: 22 (light chain CDR2), SEQ ID NO: 24 (light chain CDR3), SEQ ID NO: 26 (heavy chain CDR1), SEQ ID NO: 28 (heavy chain CDR2), SEQ ID NO: 30 (heavy chain GDR3), SEQ ID NO: 32 (light chain CDR1), SEQ ID NO: 34 (light chain CDR2), SEQ ID NO: 36 (light chain CDR3), SEQ ID NO: 38 (heavy chain CDR1), SEQ ID NO: 40 (heavy chain CDR2), SEQ ID NO: 42 (heavy chain CDR3), SEQ ID NO: 44 (light chain CDR1), SEQ ID NO: 46 (light chain CDR2), and SEQ ID NO: 48 (light chain CDR3). In addition, the polynucleotide encoding VH and VL according to the present invention is described in SEQ ID NO: 50 (VH), SEQ ID NO: 52 (VL), SEQ ID NO: 54 (VH), SEQ ID NO: 56 (VL), SEQ ID NO: 58 (VH), SEQ ID NO: 60 (VL), SEQ ID NO: 62 (VH), and SEQ ID NO: 64 (VL).

The polynucleotide encoding an antibody having a configuration of heavy and light chains according to the present invention may be specifically a nucleotide sequence selected from the group consisting of SEQ ID NO: 70 , SEQ ID NO: 72 , SEQ ID NO: 74 , SEQ ID NO: 76, SEQ ID NO: 78, SEQ ID NO: 80, SEQ ID NO: 82, and SEQ ID NO: 84. A polynucleotide encoding the heavy chain of the antibody is selected from the group consisting of SEQ ID NO: 70, SEQ ID NO: 74, SEQ ID NO: 78, and SEQ ID NO: 82; and a polynucleotide encoding the light chain of the antibody is selected from the group consisting of SEQ ID NO: 72, SEQ ID NO: 76, SEQ ID NO: 80, and SEQ ID NO: 84.

In addition, a polynucleotide encoding a fragment of the antibody may preferably contain a nucleotide sequence represented by SEQ ID NO: 66 or SEQ ID NO: 68 encoding scFv according to the present invention. Alternatively, a polynucleotide encoding a fragment of the antibody may preferably be a polynucleotide encoding Fab, which contains nucleotide sequences represented by SEQ ID NO: 58 and SEQ ID NO: 60 or nucleotide sequences represented by SEQ ID NO: 62 and SEQ ID NO: 64.

The present invention provides a recombinant expression vector including the polynucleotide encoding the antibody or fragment thereof according to the present invention.

As used herein, the term "recombinant" can be used interchangeably with "genetic manipulation", and refers to the fabrication of a gene in the form that does not exist in a natural state, using molecular cloning experiment techniques, such as gene modification, cleavage, or linkage.

As used herein, the term "expression" refers to the production of proteins or nucleic acids in cells.

As used herein, the term "recombinant expression vector" is a vector that can express a target protein or nucleic acid (RNA) in a proper host cell, and refers to a gene construct containing essential control elements that are operably linked so as to express a polynucleotide (gene) insert. The term "operably linked" refers to the functional linkage of a nucleic acid expression control sequence and a nucleic acid sequence encoding a target protein or RNA so as to perform general functions, and means the linkage therebetween so as to express a gene by an expression control sequence. The term "expression control sequence" refers to a DNA sequence that controls the expression of an operably linked polynucleotide sequence in a particular host cell. Such an expression control sequence contains a promoter for executing transcription, any operator sequence for controlling transcription, a sequence encoding a proper mRNA ribosomal binding site, a sequence for controlling the termination of transcription and translation, an initiation codon, a termination codon, a polyadenylation A signal, an enhancer, and the like.

The recombinant expression vector of the present invention is not particularly limited to a kind thereof as long as the vector is commonly used in a cloning field, and examples of the recombinant expression vector include, but are not limited to, a plasmid vector, a cosmid vector, a bacteriophage vector, and a virus vector. Examples of the plasmid may include *Escherichia* coli-derived plasmids (pBR322, pBR325, pUC118, pUC119, and pET-22b (+)), *Bacillus subtilis-derived* plasmids (pUB110 and pTP5), and yeast-derived plasmids (YEp13, YEp24, and YCp50). Examples of the virus may include: animal viruses, such as retrovirus, adenovirus, and vaccinia virus; and insect viruses, such as baculovirus. pcDNA or the like may be used.

Therefore, the recombinant expression vector according to the present invention refers to a gene construct in which a polynucleotide encoding an antibody having a configuration of CDRs, VH and VL, or heavy and light chains, which can specifically bind to PICP, is operably linked so as to be expressed in a proper host cell. Preferably, the recombinant expression vector according to the present invention includes: a polynucleotide containing a nucleotide sequence encoding a heavy chain of an antibody, selected from the group consisting of SEQ ID NO: 70, SEQ ID NO: 74, SEQ ID NO: 78, and SEQ ID NO: 82; and a polynucleotide containing a nucleotide sequence encoding a light chain of an antibody, selected from the group consisting of SEQ ID NO: 72, SEQ ID NO: 76, SEQ ID NO: 80, and SEQ ID NO: 84. In order to produce the antibody according to the present invention, it is most preferable to express a pair of polynucleotides encoding the following heavy and light chains, respectively: SEQ ID NO: 70 and SEQ ID NO: 72, SEQ ID NO: 74 and SEQ ID NO: 76, SEQ ID NO: 78 and SEQ ID NO: 80, and SEQ ID NO: 82 and SEQ ID NO: 84.

The polynucleotides encoding heavy and light chains of an antibody according to the present invention may be contained in separate recombinant expression vectors, respectively, or may be contained in one recombinant expression vector.

In addition, the recombinant expression vector according to the present invention may include a polynucleotide containing a nucleotide sequence represented by SEQ ID NO: 66 or SEQ ID NO: 68 encoding scFv. Alternatively, the recombinant expression vector according to the present invention may contain a polynucleotide encoding Fab and containing nucleotide sequences represented by SEQ ID NO: 58 and SEQ ID NO: 60 or nucleotide sequences represented by SEQ ID NO: 62 and SEQ ID NO: 64.

The present invention provides cells transfected with the recombinant expression vector including a polynucleotide encoding an antibody or fragment thereof according to the present invention.

The cells of the present invention are not particularly limited to the kind thereof as long as the cells can be used to express a polynucleotide encoding an antibody or fragment thereof contained in the recombinant expression vector of the present invention. The cells (host cells) transfected with the recombinant expression vector according to the present invention may be prokaryotic cells (e.g., *E. coli*), eukaryotic cells (e.g., yeast or other fungi), plant cells (e.g., tobacco or tomato plant cells), animal cells (e.g., human cells, monkey cells, hamster cells, rat cells, mouse cells, or insect cells), or hybridomas derived therefrom. Preferably, the cells may be derived from mammals including humans. For example, HEK293F cells or the like may be used.

The recombinant expression vector according to the present invention, which can express a polypeptide of an antibody or fragment thereof specifically binding to PICP, can be introduced in cells for producing an antibody or fragment thereof for transfection, by a method known in the art, such as, but is not limited to, transient transfection, microinjection, transduction, cell fusion, calcium phosphate precipitation, liposome-mediated transfection, DEAE dextran-mediated transfection, polybrene-mediated transfection, electroporation, gene gun, and other methods well known in the art to induce nucleic acids into cells. The cells transfected with the recombinant expression vector according to the present invention produce heavy and light chain of the antibody according to the present invention or a fragment of the antibody.

Furthermore, the present invention provides a method for producing an antibody or fragment thereof specifically binding to PICP, the method including:
(a) transfecting host cells with the recombinant expression vector;
(b) culturing the transfected host cells to produce an antibody or fragment thereof; and
(c) harvesting the antibody or fragment thereof produced in the host cells.

In step (a), in order to produce the antibody or fragment thereof according to the present invention, host cells are transfected with the recombinant expression vector, in which the polynucleotide encoding the antibody or fragment thereof is operably linked.

A person skilled in the art may execute the present step by selecting a proper transfection method according to the selected host cells and recombinant expression vector as described above. Most preferably, a recombinant expression vector is selected from recombinant expression vectors containing a pair of nucleotide sequences encoding heavy and light chains represented by SEQ ID NO: 70 and SEQ ID NO: 72; SEQ ID NO: 74 and SEQ ID NO: 76; SEQ ID NO: 78 and SEQ ID NO: 80; or SEQ ID NO: 82 and SEQ ID NO: 84, for transfection of host cells. Of the selected pair of nucleotide sequences, the recombinant expression vector including nucleotide sequences of heavy and light chains can be co-transfected in the same host cell to allow the heavy and light chains to be expressed in one cell, or the recombinant expression vector including nucleotide sequences of heavy and light chains can be transfected in separate host cells to allow the heavy and light chains to be separately expressed.

In step (b), the transfected host cells are cultured to produce polypeptides of heavy and light chains of the antibody or a fragment of the antibody according to the present invention from the recombinant expression vector introduced into the host cells.

The medium composition, culture conditions, and culture time for culturing the host cells may be appropriately selected according to a method commonly used in the art. The antibody molecules produced in the host cell may be accumulated in the cellular cytoplasm, may be secreted outside the cell or in the culture medium by a suitable signal sequence, or may be targeted using a periplasm or the like. It is also preferred that the antibody according to the present invention has a functional confirmation through protein refolding using a method known in the art so as to maintain binding specificity for PICP. In addition, for the production of IgG type antibody, heavy and light chains are expressed in separate cells, and then contacted with each other in a separate step to constitute the whole antibody, or heavy and light chains are expressed in the same cell and form the whole antibody inside the cell.

In step (c), the antibody or fragment thereof produced in the host cells is obtained. A person skilled in the art can properly select and control the obtaining method in consideration of characteristics of the antibody or fragment polypeptide thereof produced in the host cells, characteristics of the host cells, the mode of expression, or the targeting or not of the polypeptide. For example, the antibody or fragment thereof secreted into the culture medium can be collected by obtaining the culture medium of the host cells and performing centrifugation to remove impurities. In order that the antibody present in specific organelles or cytoplasm in the cell are released and collected to the outside of the cell, the cell may be lysed within an extent that does not affect the functional structure of the antibody or the fragment thereof. Furthermore, the obtained antibody may be further subjected to impurity removal and concentration processes through chromatography, filtration using a filter, dialysis, or the like.

Furthermore, the present invention provides a method for producing a recombinant PICP protein, the method including:
(i) constructing recombinant expression vectors including polynucleotides encoding PICP α1 chain and PICP α2 chain, respectively, a signal peptide stimulating extracellular secretion and a label protein being conjugated to each of the chains;
(ii) co-transfecting cells with a mixture of the recombinant expression vector including the polynucleotide encoding PICP α1 chain and the recombinant expression vector including the polynucleotide encoding PICP α2 chain;
(iii) culturing the co-transfected cells; and
(iv) obtaining PICP protein produced in the cells.

The present inventors successfully produced recombinant PICP protein using an antigen for developing an antibody specifically binding to the trimeric protein PICP. The present inventors confirmed that procollagen α1 and α2 chain polypeptides constituting PICP are co-expressed in the same cell to form a heterotrimer of procollagen α1 and α2 polypeptides at a ratio of 2:1 like in natural-state PICP, and therefore, there is provided an improved method for producing recombinant PICP protein, whereby PICP protein can be obtained at high purity and high efficiency.

PICP is a protein that is composed of a complex of three types of proteins and is generated from procollagen through cleavage by a protease action in a natural state, and thus it is difficult to predict whether PICP has a similar three-dimensional structure similar to the configuration of polypeptides in a natural state when PICP is fabricated as a recombinant protein. An attempt to express PICP alone using a recombinant protein or an accurate three-dimensional structure of PICP has not been reported.

So far, a major method of obtaining PICP protein has been to isolate and purify naturally produced PICP in cells or in the body. In addition, it is difficult to remove the contamination of PIIICP, which is very similar to PICP in size and structure and is present at a high level next to PICP, in the isolation of PICP. Therefore, a purification method using a difference of mannose rich oligosaccharide side chain between PICP and PIIICP, a method of first cleaving PICP procollagen and then cleaving PICP using a recombinant enzyme, and a method of obtaining PICP secreted in the culture supernatant of human fibroblast expressing collagen at a high level are supposed. However, these methods have many problems in that a cleavage enzyme for recombinant PICP produced in bacteria has low accuracy, causing a modification at the terminals of PICP, or the yield of PICP is very low.

As used herein, the term "protein" is used interchangeably with the term "polypeptide" or "peptide", and refers to, for example, a polymer of amino acid residues, as typically found in proteins in nature. In addition, the term "fragment" refers to a portion of a protein.

As used herein, the term "polynucleotide" or "nucleic acid" refers to single- or double-stranded deoxyribonucleotide (DNA) or ribonucleotide (RNA) Unless otherwise limited, the term includes known analogs of naturally occurring nucleotides that hybridize with nucleic acids in a manner similar to naturally occurring nucleotides. The term "mRNA" refers to RNA that transfers genetic information of the nucleotide sequence of a specific gene to a ribosome forming a polypeptide during protein synthesis.

In step (i) in the method for producing a recombinant PICP protein, recombinant expression vectors including polynucleotides encoding PICP α1 and PICP α2 chains are constructed, wherein a signal peptide stimulating extracellular secretion and a label protein are conjugated to each of the chains. The PICP α1 and PICP α2 chains mean polypeptides corresponding to portions constituting PICP in procollagen α1 and α2 chains, respectively. The recombinant expression vectors mean gene constructs in which polynucleotides encoding PICP α1 and PICP α2 chains operably linked to essential control elements, such as expression a control sequence, a signal peptide stimulating extracellular secretion and a label protein being conjugated to each of the chains, such that recombinant PICP protein can be synthesized in host cells and secreted out of the cells (that is, into cell culture). Any known label protein that is useful in the detection or isolation of PICP α1 and α2 chains can be used as the label protein without limitation, and for example, His, C-myc, and the like may be used. The PICP α1 and α2 chain proteins are preferably conjugated to different types of label proteins, respectively. In addition, PICP α1 and α2 chain proteins are conjugated to signal peptides inducing extracellular secretion such that the PICP α1 and α2 chain proteins can be easily obtained, and for example, the murine Ig k-chain V-J2-C signal peptide or the like may be used. The amino acid sequences of PICP α1 and α2 chain proteins including the murine Ig k-chain V-J2-C signal peptide and His (PICP α1 chain) or C-myc tag (PICP α2 chain) at the N-terminal, as specific examples of the PICP α chain proteins, to each of which a signal peptide stimulating extracellular secretion and a label protein are conjugated, in step (i), are described in SEQ ID NO: 111 and SEQ ID NO: 112, respectively. A person skilled in the art can construct polynucleotides encoding PICP chains, to each of which a signal peptide and a label protein are conjugated, and recombinant expression vectors including the polynucleotides.

The polynucleotides encoding PICP α1 and α2 chains may be preferably derived from mammals including humans, and most preferably may contain nucleotide sequences represented by SEQ ID NO: 86 and SEQ ID NO: 88, respectively. The amino acid sequences of PICP α1 and α2 chains encoded by the nucleotide sequences are described in SEQ ID NO: 85 and SEQ ID NO: 87, respectively.

The recombinant expression vectors constructed in step (i) are co-transfected in the same host cell.

Preferably, the recombinant expression vectors including polynucleotides encoding PICP α1 and α2 chains, constructed in step (i), are simultaneously introduced into the host cell, at a molar ratio of 2: 1, that is, a mixture of one molecule of the recombinant expression vector of PICP α1 chain and two molecules of the recombinant expression vector of PICP α2. PICP α1 and α2 chain proteins expressed in the same host cell are combined at a compositional ratio of 2:1 to form PICP protein.

The transfection of the host cell can be carried out by properly selecting an intercellular polynucleotide delivery method known in the art. Examples of the method may include *in vitro* transfection, injection or microinjection, dectroporation, heat shock, protoplast fusion, calcium phosphate (CaPO₄) precipitation, calcium chloride (CaCl) precipitation, silicon carbide whiskers, shaking with silicon carbide fibers, sonication, transfection with liposomes, receptor-mediated transfection, Agrobacterium-mediated transformation, precipitation with polyethylene glycol, methods using polyethyleneimine or dextran sulfate, a method using lipofectamine, microparticle bombardment, particle gun bombardment, and the like, but are not limited thereto.

In step (iii), the co-transfected cells are cultured for a sufficient time to produce recombinant PICP protein.

A person skilled in the art can properly select the time and culture conditions sufficient for expression of the recombinant PICP. In the culture step, the PICP protein can be detected by time to check the synthesis efficiency.

In step (iv), the recombinant PICP protein produced in the co-transfected cells is obtained.

A person skilled in the art can properly select and optimize a method for obtaining a recombinant protein produced in a host cell, in consideration of characteristics of the host cell, the mode of expression of the recombinant expression vectors (for example, characteristics of promoter), the targeting or not of the polypeptide, extracellular secretion, or the like. The recombinant protein secreted in the culture medium by the extracellular secreting signal peptide conjugated in step (i) may be collected by obtaining the culture medium of the host cell and then removing impurities through centrifugation. In order to collect the recombinant protein remaining in specific organelles or cytoplasm in the cell as needed, the cell may be lysed by a method that does not affect the functional structure of the protein. Furthermore, the obtained recombinant protein may further pass through impurity removal and concentration processes through chromatography, filtration using a filter or the like, dialysis.

The production of PICP protein by a gene recombinant technique as above can prevent a variation of PICP that may occur from abnormal activity of a recombinant enzyme when the isolated procollagen is treated with the recombinant enzyme, can easily obtain high-purity protein products without other containments by binding a particular tag to the recombinant PICP protein, and can obtain a higher yield in comparison with when the naturally occurring PICP protein is obtained.

### Advantageous Effects

The protein complex PICP can be easily produced at high purity and high efficiency by using the method of the present invention. Furthermore, the antibody or fragment thereof according to the present invention has very high binding affinity and binding specificity and no cross-reactivity to similar polypeptides, and the amino acid sequences of the sites of the antibody or fragment thereof, which are important for antigen recognition and binding, are specified, so that the antibodies can be easily mass-produced repeatedly.

### Brief Description of the Drawings

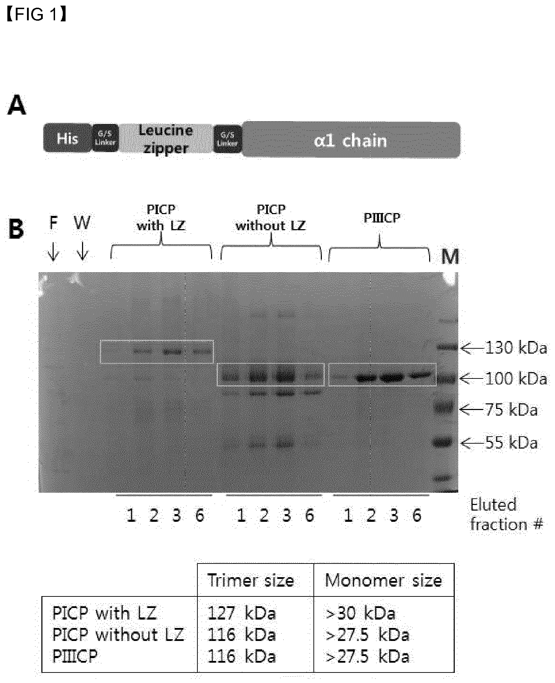

FIG. 1 shows the results of preliminary experiments for expressing recombinant PICP antigen FIG. 1A is a schematic diagram of PICP α1 chain with leucine zipper domain introduced thereinto. FIG. 1B shows a Coomassie stained SDS-PAGE gel image to confirm the expression tendency of PICP antigen containing PICP α1 added with leucine zipper domain. In the gel image, the squares indicate bands expected to be a protein complex. In the image, LZ represents leucine zipper and M represents marker. F represents the buffer eluted while a resin bound to protein is loaded onto the column, and W represents the buffer eluted by column washing.
FIG. 2 is a schematic diagram of recombinant expression vectors for expressing recombinant PICP antigens. Pcmv represents a promoter, and SP represents a signal peptide (murine Ig K-chain V-J2-C signal peptide).
FIG. 3 shows western blot results of confirming recombinant PICP antigen proteins expressed in HEK293F cells.
FIG. 4 shows the SDS-CGE and bioanalyzer analysis results of confirming PICP antigen proteins expressed in HEK293F cells. FIG. 4A shows SDS-CGE results of recombinant PICP or PIIICP protein. FIG. 4B shows the SDS-CGE and bioanalyzer results of a marker (ladder). FIGS. 4C and 4D show bioanalyzer analysis results in non-reducing or reducing conditions of PICP and PIIICP.
FIG. 5 shows indirect ELISA results for selecting scFv specifically binding to recombinant PICP. FIG. 5A indicates a ratio of ELISA signal using PICP and ELISA signal using PIIICP (ratio of O.D. 450nm; PICP/PIIICP). FIG. 5B indicates a ratio of ELISA signal using PICP and ELISA signal using a control without antigen (PICP/control). FIG. 5C indicates a ratio of ELISA signal using PIIICP and ELISA signal using a control without antigen (PIIICP/control).
FIG. 6 shows indirect ELISA results for confirming the biding affinity of 2D and 4G scFv to antigens. FIG. 6A and FIG. 6B show ELISA results using PICP and PIIICP as antigens, respectively.
FIG. 7 shows SDS-CGE and bioanalyzer analysis results for confirming 2D and 4G IgG expressed in 293F cells. FIG. 7A shows SDS-CGE results of IgG. FIG. 7B shows the SDS-CGE and bioanalyzer results of a marker (ladder). FIGS. 7C and 7D show bioanalyzer assay results in non-reducing or reducing conditions of 2D and 4G IgG, respectively.
FIG. 8 shows indirect ELISA results for confirming the biding affinity of 2D and 4G IgG to antigens. FIG. 8A and FIG. 8B show ELISA results using PICP and PIIICP as antigens, respectively.
FIG. 9 shows sandwich EILSA results using 2D, 4G, clone 1, and clone 2 IgG. FIG. 9A and FIG. 9B show ELISA results using PICP and PIIICP as antigens, respectively.
FIG. 10 shows a standard curve of sandwich ELISA using 2D IgG.
FIG. 11 shows sandwich ELISA results for confirming cross-reactivity of 2D IgG to PIIICP.
FIG. 12 shows the results of quantitatively confirming binding affinity of 2D IgG to PICP through SPR method.
FIG. 13 shows the results of quantitatively confirming binding affinity of 4G IgG to PICP through SPR method.

### Mode for Carrying Out the Invention

Hereinafter, the present invention will be described in detail.

However, the following examples are merely for illustrating the present invention, and are not intended to limit the scope of the present invention.

### <Example 1>

### Fabrication of recombinant PICP antigen

### <1-1> Construction of expression vectors for expressing recombinant PICP antigen

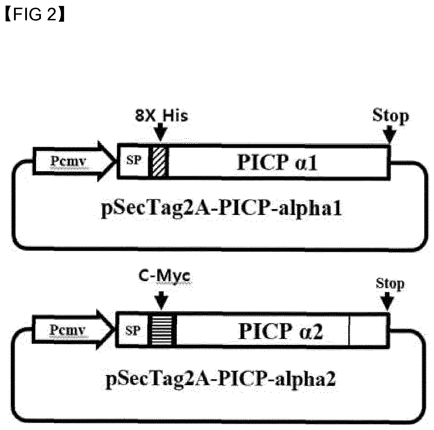

Expression vectors capable of expressing a recombinant PICP protein as an antigen protein for constructing antibodies specific to PICP were constructed. The structures of recombinant expression vectors for expressing recombinant procollagen α1 chain (PICP α1 chain) and α2 chain (PICP α2 chain) constituting PICP are shown in FIG. 2. In order to investigate whether expressed PICP accurately formed a trimeric structure after PICP constituent proteins were purified, 8xHis (histidine) tag was conjugated to the N-terminal of α1 chain and c_Myc tag was conjugated to the N-terminal of α2 chain. Further, the murine Ig K-chain V-J2-C signal peptide as a signal peptide (SP) inducing a protein to secrete out of cells was conjugated to each N-terminal. Polynucleotides encoding α1 chain and α2 chain, to which the signal peptide and His or the signal peptide and Myc tag were conjugated, were inserted into pSecTag2A expression vectors.

For construction of the recombinant expression vectors, the PICP portions in the vectors including procollagen 1 α1 chain and α2 chain genes were amplified using PCR. The PICP α1 chain gene was amplified into one PCR product using the following primers: 5'-AAAAGGCGCGCCCATCACCATCACCATCACCATCACGGCGCCGATGATGCCAATGT GGTTCG-3' (forward, SEQ ID NO: 91); 5'-TTGGGCCCTTACTACAGGAAGCAGACAGGGC-3' (reverse, SEQ ID NO: 92). The PICP α2 chain gene was amplified such that the PICP α2 chain was divided into two portions, the N-terminal fragment and the C-terminal fragment, followed by separating amplification, and then the respective two amplified portions are linked using overlap extension PCR, thereby attaining the amplification of the gene of complete PICP α2 chain. Since the PICP α2 gene contains a recognition site for the Apal restriction enzyme to be used for cloning, a point mutation for eliminating the Apal recognition site was introduced into the primer for amplifying the PICP α2 gene (adenine marked in bold type in the N-terminal fragment reverse primer). The nucleotide sequences of primers for amplifying PICP α2 chain gene were as follows: 5'-AAAAGGCGCGCCGAACAGAAACTGATCTCTGAAGAAGACCTGGGCGCCGACCAGCC TCGCTCAG-3' (N-terminal fragment, forward, SEQ ID NO: 93); 5' -GGATGTTTTCAGGTTGGGC**A**CGGATACAGGTTTCGCC-3' (N-terminal fragment, reverse, SEQ ID NO: 94); 5'-GCCCAACCTGAAAACATCC-3' (C-terminal fragment, forward, SEQ ID NO: 95); 5'-TTGGGCCCCTATTATTTGAAACAGACTGGGCCAATG-3' (C--terminal fragment, reverse SEQ ID NO: 96). For the overlap extension PCR, the forward primer (SEQ ID NO: 93) for the N-terminal fragment and the reverse primer (SEQ ID NO: 96) for the C-terminal fragment were used. All the primers were fabricated by Macrogen (Korea).

The PICP α1 chain gene was constructed by performing polymerase chain reaction (PCR) using pfu polymerase (Nanohelix, Korea) (PCR cycle: 30 cycles of 95□ for 30 seconds , 53□ for 30 seconds, and 72□ for 52 seconds). In the PICP α2 chain gene, the two respectively amplified PCR products were electrophoresed on 1% agarose gel, and purified and isolated using an agarose gel purification kit (Intron, Korea), and then overlap extension PCR was performed in the same PCR conditions, thereby finally fabricating a PICP α2 chain gene product.

The fabricated PICP α1 and α2 chain PCR products were inserted into pSecTag2A vector (Invitrogen) as animal cell expression vectors using Ascl/Apal restriction enzyme, thereby constructing pSecTag2ArPICP α1 vector and pSecTag2A_PICP α2 vector.

In addition, vectors for expressing procollagen type III C-terminal propeptide (PIIICP) as a comparative antigen compared with PICP were constructed by a similar method.

PIIICP, compared with PICP, has similar constitution proteins, forms a very similar heterotrimeric structure, and is present in large quantities next to PICP in the body. Therefore, the cross-reactivity with PIIICP needs to be checked in determining of specificity of PICP antibodies. His tag was conjugated to the PIIICP protein. The nucleotide sequences of the primers used to amplify the PIIICP gene were as follows: 3'-AAAAGGCGCGCCCATCACCATCACCATCACCATCACGGCGCCGATGAACCAATGGA TTTCAAAATC-5' (forward, SEQ ID NO: 97), 3'-TTGGGCCCCTATTATAAAAAGCAAACAGGGCCAAC-5' (reverse, SEQ ID NO: 98).

### <1-2> preliminary experiments for efficiency in construction of recombinant PICP antigen

PICP is a heterotrimer of proteins combined with two procollagen type 1 α1 chains and one two procollagen type 1 α2 chain. PICP is generated by cleavage of the C-terminal site of procollagen in a natural state, and thus it is difficult to predict whether PICP has a similar three-dimensional structure similar to the configuration of polypeptides in a natural state when PICP is fabricated as a recombinant protein. In addition, a preparation of PICP alone as a recombinant protein or an accurate three-dimensional structure of PICP has not been reported.

Therefore, the strategy of introducing leucine zipper (LZ) domain into the PICP α1 chain to form a normal trimeric structure when only the polypeptide portion (hereinafter, referred to as PICP α1 chain or PICP α2 chain) constituting PICP in procollagen was expressed as a recombinant protein was attempted (FIG. 1A). The LZ domain is used in the production of dimers or trimers of proteins in the field of protein engineering. It was predicted that, first, the action of the leucine zipper introduced into the PICP α1 chain stimulates the formation of homodimers of the PICP α1 chain, and sequentially the PICP α2 chain is allowed to bind to the PICP α1 chain homodimer to form a PICP trimer.

In order to investigate the expression ratio of the PICP recombinant protein with LZ domain introduced thereinto, a nucleotide sequence encoding the GCN4 leucine zipper domain was added to the pSecTag2A-PICP α1 vector including PICP α1 chain, constructed in example <1-1>, so that the domain was conjugated to the N-terminal site of PICP α1 chain.

The pSecTag2A-PICP α1 vector and the pSecTag2A-PICP α2 vector with or without LZ were mixed at a molar ratio of 2:1, and co-transfected into HEK293F cells. The transfected cells were cultured for 7 days, and culture supernatants were harvested, and recombinant PICP was purified using Ni/NTA resin (GE, USA). 1.5 ml of the Ni/NTA resin per 50 ml of the supernatant was added, and homogeneously mixed in a 50 ml-tube, followed by addition of imidazole (final concentration of 10 mM), followed by rotation at 4 rpm for 1 hour and 30 minutes at 4□, so that PICP was immobilized onto the resin. The column was washed with 50 ml of PBS (20mM phosphate, 137mM NaCl, 2.7mM KCl, pH 7.4, 20mM imidazole) containing imidazole (20 ml), and then eluted with PBS (20mM phosphate, 137mM NaCl, 2.7mM KCl, pH 7.4, 20mM imidazole) containing imidazole (20 ml). Eluted fractions were electrophoresed on SDS-PAGE under non-reducing conditions, and the protein expression tendency was investigated through Coomassie staining. PIIICP was also expressed, isolated, and purified by the same methods.

As shown in FIG. 1B and Table 1, in the cells in which the PICP α1 and PICP α2 chains bound to the leucine zipper domain were expressed (PICP with LZ), the yield of protein was unexpectedly small, and the bands of proteins, which were difficult to explain by the molecular weight of the proteins expected from the PICP trimer, were defined. Rather, the proteins expected as the trimeric PICP were expressed at high levels in the cells in which the PICP α1 and PICP α2 chains containing no leucine zipper domain were expressed (PICP without LZ) .

**[Table 1]**

| Recombinant protein expression efficiency | | |
|---|---|---|
| **Sample** | **Concentration (BCA assay)** | **Yield (100 ml Culture basis)** |
| PICP without LZ | 130 µg/ml | 1.402 mg |
| PICP with LZ | 81.8 µg/ml | 1.145 mg |
| PIIICP | 96 µg/ml | 1.536 mg |

### <1-3> Expression and purification of recombinant PICP antigens

As confirmed in example <1-2>, expression vectors including nucleotide sequences encoding recombinant PICP α1 and α2 chains without LZ were expressed in animal cells, thereby producing and purifying recombinant PICP antigen proteins.

The pSecTag2A-PICP α1 vector and the pSecTag2A-PICP α2 vector were mixed at a molar ratio of 2:1, and co-transfected into HEK293F cells. The co-transfected cells were cultured for 7 days, and culture supernatants were harvested, and the proteins were isolated and purified by the same method as in example <1-2>. PIIICP protein was also expressed, isolated, and purified by the same method. The sizes and purities of the purified proteins were analyzed using SDS-PAGE and western blotting (anti-His tag, anti-c-Myc tag) in reducing or non-reducing conditions.

The yields of the recombinant PICP proteins obtained from the culture supernatants by the method were about 1 mg per 100 ml of the culture supernatant, and these yields were greatly higher than the previously reported yields (1-3 mg/1 L) when naturally occurring PICP proteins were obtained (EP 0465104 B1; US 5,698,407 ; Pedersen BJ et al., Clin Chem, 40(5):811-816, 1994) .

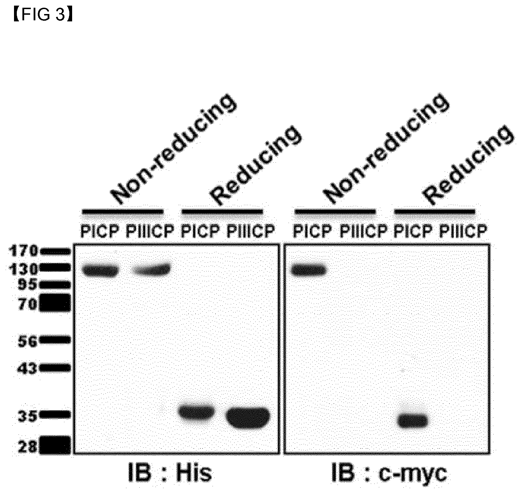

As shown in FIG. 3, in a case of electrophoresis in reducing conditions, the binding of the complex composed of recombinant PICP α1 and α2 chains was dissociated, and thus single protein bands near a molecular weight marker 35 kDa were detected by His and Myc antibodies, respectively. A 35-kDa single band was observed in western blotting using an antibody binding to His included in the PICP α1 chain, and a 32-kDa single band was observed in western blotting using an antibody binding to Myc included in the PICP α2 chain. It was confirmed that the PIIICP prepared as a comparative antigen to PICP was also expressed at an expected molecular weight.

Meanwhile, in a case of electrophoresis in non-reducing conditions, 118-kDa single protein bands were detected in both of western blotting using His antibody and Myc antibody, and thus PICP configured a trimer, expectedly.

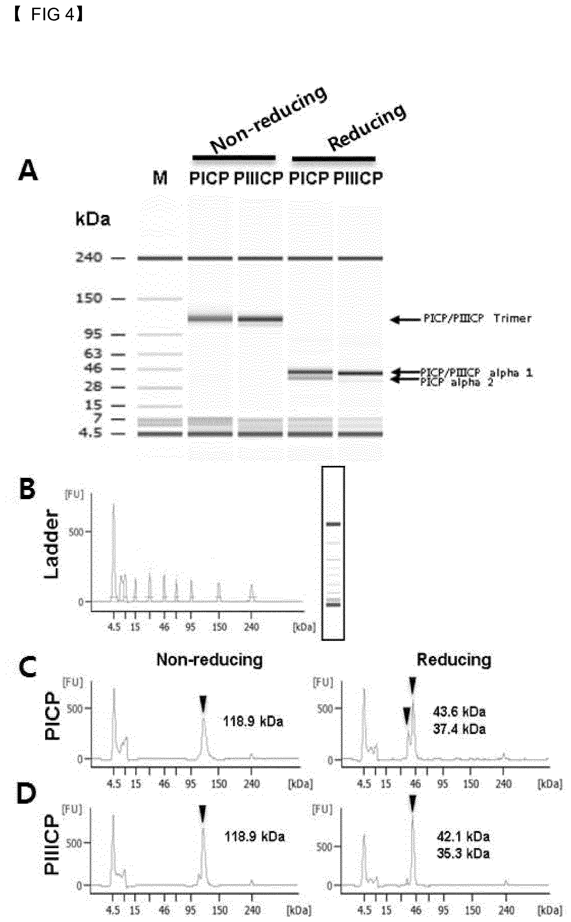

In addition, as shown in FIG. 4, it was also confirmed that the recombinant PICP antigens were expressed as proteins having expected molecular weights (118.9 kDa for complex in non-reducing conditions; 43.6 kDa for PICP α1 chain in reducing conditions; and 37.4 kDa for PICP α2 chain in reducing conditions) in the protein analyses using SDS-CGE (capillary gel electrophoresis) and Bioanalyzer (Agilent 2100 Bioanalyzer). Specific Bioanalyzer analysis results are shown in Table 2. In addition, the total concentrations (amino acid concentrations) of PICP α1 and α2 chains with similar protein sizes were 67.5% and 30.5%, respectively, confirming that PICP α1 and α2 constituted the protein complex of PICP at a ratio of 2: 1, like in the natural state.

**[Table 2]**

| **Antigen protein bioanalyzer analysis results** | | | |
|---|---|---|---|
| **Antigen** | **Total Rel. Conc. (ng/µl)** | | **Rel. Conc. (ng/µl)** |
| **PICP** | **Non reducing** | **1,164 (118.9 kDa)** | **1,143(98.2%)** |
| | **Reducing** | **2,411 (43.6;37.4 kDa)** | **1,625(67.4%)** |
| | | | **734(30.5%)** |
| **PIIICP** | **Non reducing** | **1,727 (118.9 kDa)** | **1,518(87.9%)** |
| | **Reducing** | **2,323 (42.1 kDa)** | **2,162(93.1%)** |

### <Example 2>

### scFv library screening

### <2-1> scFv phage selection

In order to select scFv specifically binding to the recombinant PICP antigen proteins prepared in <Example 1>, the phage display panning experiment was executed using scFv phage library derived from human B cells and labeled with HA tag. The phage library used in the example is described in Korean Patent No. 10-0961392.

First, 1-10 ug of antigen proteins were added to immuno-tubes containing 1 ml of 1X PBS solution, followed by incubation at 37□ for 1 hour at 200 rpm, so that the antigen was coated on inner surfaces of the tubes. Antigen solutions were drained and washing was conducted once with tap water, thereby removing uncoated antigen. In order to prevent non-specific binding between antigen proteins and phages, the immune-tubes and scFv libraries were separately incubated with 1X PBST (0.05% tween20-containing PBS) containing 3% skim milk at room temperature for 1 hour. After the skim milk was removed from the immuno-tubes, scFv libraries were added, followed by incubation at 150 rpm for 1 hour at 37□, so that scFv phages were bound to PICP antigens. In order to find scFv specifically binding to procollagen type 1, about 1-5 ug of procollagen type 3 was added to reaction solution. The scFv phages were incubated in the tubes, and then washed two or five times with 1X PBST, thereby removing unbound scFv phages.

ScFv phages specifically binding to recombinant PICP antigens were isolated within 10 minutes by addition of 1 ml of triethylamine (100 mM) at room temperature, and neutralized with Tris (1 M, pH 7.4). The filtered phage scFv was added to ER2537 *E. coli* cultured to OD<1, followed by infection with incubation at 120 rpm for 1 hour 30 minutes at 37°C. *E. coli* infected with the phages was centrifuged to partially remove culture supernatant, and then coated on a 15 cm-diameter agarose plate containing ampicillin and glucose (2%) through re-dispersion. The next day, 5 ml of SB medium was applied to obtain all of the cells grown on the plates, and glycerol (50%) was added to have 0.5 times of the total volume, followed by mixing, and then 1 ml was dispensed for each and stored at -80□ (scFv panning stock). 20 µl of the prepared stock was inoculated in 20 ml of SB solution, followed by incubation, and helper phages were used to construct scFv phage - library (1 ml) for the next step of phage panning. The above process for isolating phages expressing scFv specific to PICP antigens was repeated two or three times.

### <2-2> Selection of scFv antibodies specifically binding to PICP

Indirect ELISA using PICP and PIIICP as antigens was conducted to investigate whether the scFv expressed in the phages selected in Example <2-1> specifically binds to recombinant PICP antigen proteins.

The scFv products obtained by 3 rounds of panning were diluted, and coated on 10 cm-diameter agarose plates. The next day, colons for each were selected, and were incubated in 96-well plates containing 200 µl of SB medium. It was confirmed that growth was good overall, and IPTG (1 mM) was added, followed by incubation at 30□ for 16 hours, thereby inducing the production of scFv. The next day, the 96-well plates were centrifuged to isolate only cells, and then the cells were lysed with TES solution, followed by centrifugation, thereby isolating only supernatants. The obtained supernatants were subjected to indirect ELISA to select scFv specifically binding recombinant PICP antigens. The plates were coated with PICP or PIIICP prepared in the previous example, incubated with culture supernatants containing scFv, and then incubated with anti-HA-HRP antibodies (Roche Applied Science) as secondary antibodies. A color development reaction was conducted using tetramethyl benzimidine (TMB, Thermo scientific), and stopped by using H₂SO₄ (1 M), and the absorbance was read at 450 nm using ELISA reader. The absorbance determined in the experiment using PICP antigen was calculated as a ratio relative to the absorbance determined in the experiment using PIIICP or the control experiment ((PICP/PIIICP or PICP/control), and the calculation value was used as an index for selecting scFv.

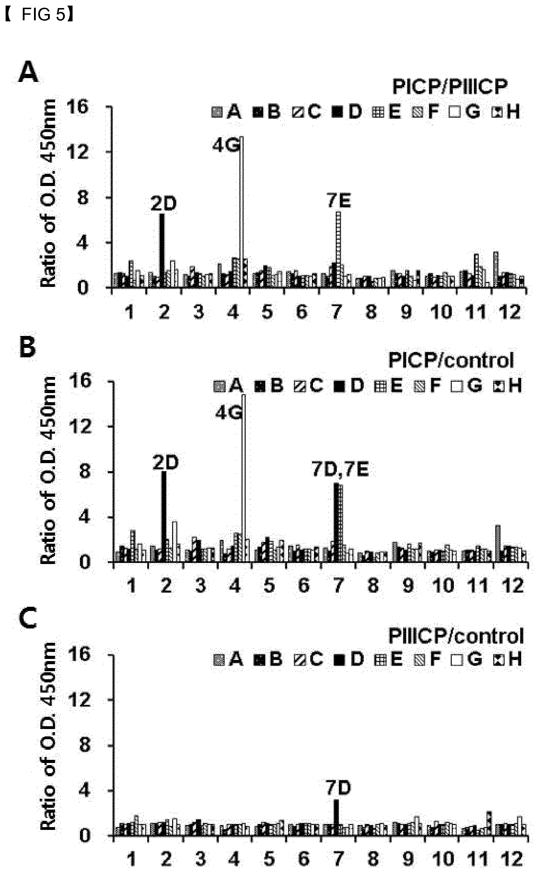

As shown in FIG. 5, the scFv expressed in colons 2D, 4G, and 7E showed higher PICP absorbance values than PIIICP (FIG. 5A, PICP/PIIICP), and thus the scFv was determined as reacting differentially with PICP and PIIICP and specifically binding to PICP antigens. Of these, scFv in clone 7E is relatively low in the absorbance value of PICP compared to control (FIG. 5B, PICP/control), determining that background signals due to the non-specific reaction were high. Therefore, 2D and 4G were selected as phage clones for producing PICP-specific scFv.

### <2-2> Verification of scFv antibodies specifically binding to PICP

The antigen binding affinity and biding specificity of scFV of clones 2D and 4G were investigated using indirect ELISA.

PICP and PIIICP produced in the previous example as antigens were immobilized at a concentration of 2 ug/ml for each in microtiter plates at room temperature for 1 hour, and blocked with PBST containing 2% skim milk for 1 hour at room temperature. 2D and 4G scFv clones (referred to as 2D scFv and 4G scFv, respectively), which were sequentially diluted within the range of 30 to 2000 ng/ml, and were added to the blocked plates at 100 µl per well, followed by incubation at room temperature for 1 hour. Anti-HA-HRP antibodies (1: 2,000) as secondary antibodies were added at 10 µl per well, followed by incubation at room temperature for 1 hour. The microplates incubated with antibodies were washed three times or four times with PBST, and then TMP solution was added at 50 µl per well to conduct a color development reaction at room temperature for 5-10 minutes. Thereafter, the reaction was stopped with H₂SO₄ (1 M), and the absorbance was read at 450 nm using ELISA reader.

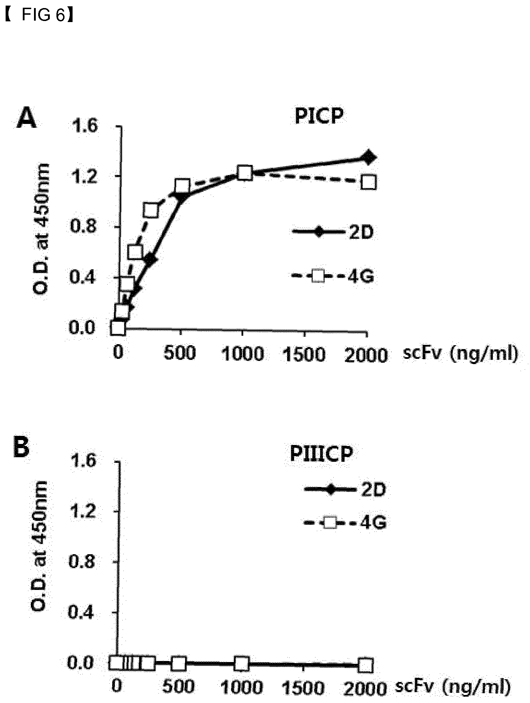

As can be confirmed in FIG. 6, it was observed that 2D scFv and 4G scFv sensitively bound to PICP antigens at lower concentration and reactions were saturated at a scFv concentration of 500 mg/ml or more (FIG. 6A). 4G scFv was slightly stronger that 2D scFv in the antigen binding affinity to PICP. Meanwhile, 2D scFv and 4G scFv never did not bind to PIIICP, and thus showed very excellent binding specificity to PICP (FIG. 6B).

### <2-4> Conversion of scFv antibody into IgG

The previously selected 2D and 4G scFv fragments were converted into IgG form, which is a more commonly used antibody.

First, polynucleotides encoding scFv were amplified from 2D and 4G clone phages by PCR. The nucleotide sequences of primers used to amplify the genes of VH regions of 2D and 4G scFv fragments were as follows: 5'-AGAGAGTGTACACTCCCAGGCGGCCGAGGTGCAGCG-3' (forward, SEQ ID NO: 99); 5'-CGCCGCTGGGCCCTTGGTGGAGGCTGAGCTCACGGTGACCAG-3' (reverse, SEQ ID NO: 100). The nucleotide sequences of primers used to amplify the genes of VL regions of 2D and 4G scFv fragments were as follows: 5'-AAGCGGCCGCCACCA TGGGATGGAGCTGTATCATCCTCTTCTTGGTAGCAACAGCTACAGGTGTACACTCC CAGTCTGTGCTGACTCAG-3' (forward, SEQ ID NO: 101); 5'-CGCCGCCGTACGTAGGACCGTCAGCTTGGT-3' (reverse, SEQ ID NO: 102). PCR was performed with 2D or 4G clone phage DNA (50ng) as a template using the primers (10 pmol for each) in conditions of: 95□ / 3 min; 95□ / 30 sec, 60□ / 30 sec, 72□ / 30 sec, 30 cycles; and 72□ / 5 min, thereby amplifying 2D or 4G scFv VH or VL gene. The PCR product was inserted into the pcDNA3.4 vector used in the production of IgG using restriction enzymes. Heavy and light chain proteins of IgG were individually expressed in separate plasmids.

The vectors containing DNA encoding heavy chain and light chain of IgG including variable regions of the constructed 2D or 4G scFv (hereinafter, referred to as 2D IgG and 4G IgG) were co-transfected into freestyle 293F cells, so that the heavy chain and light chain were expressed together in cells. The transfected 293F cells were cultured in conditions of 37°C and 8% CO₂ for 7 days, and supernatant was obtained. The supernatant was filtered through a cellulose acetate membrane filter (pore size 0.22 m, Corning), and purified using a CaptivAlM PriMAB protein A column (Repligen, USA). The concentrations of the obtained antibodies were measured using BCA kit (Pierce, 23225), and the IgG antibody proteins produced in reducing and non-reducing conditions were analyzed.

**[Table 3]**

| **IgG bioanalyzer analysis results** | | | |
|---|---|---|---|
| **antibody** | **Total ReL Conc. ng/ul** | | **Rel. Conc. ng/ul(%)** |
| **2D** | **Non reducing** | **85.4 (170.8 kDa)** | **83.1(70.9%)** |
| | **Reducing** | **671.3 (60,8.26,3 kDa)** | **349.3(61.1%)** |
| | | | **197.7(34.6%)** |
| **4G** | **Non reducing** | **64.3 (169.6 kDa)** | **63.9(83.8%)** |
| | **Reducing** | **893.2 (62;26.8 kDa)** | **498(556,8%)** |
| | | | **316.6(35.3%)** |

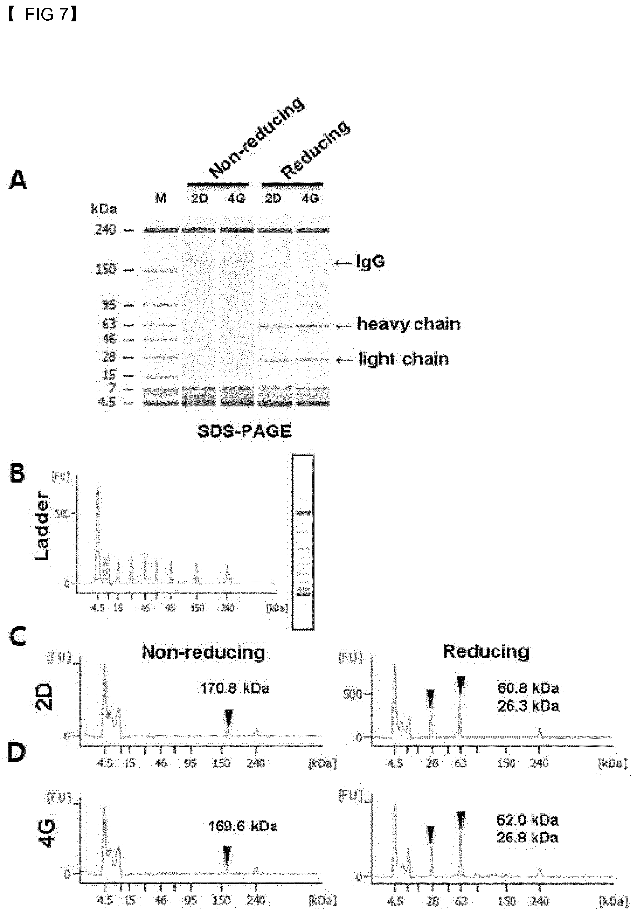

As shown in FIG. 7, it was confirmed that all the heavy and light chains of 2D IgG and 4G IgG were well expressed with expected molecular weights. Specific bioanalyzer analysis results are shown in Table 3. In non-reducing conditions, 2D IgG and 4G IgG were showed to have molecular weights of 170.8 kDa and 169.6 kDa, respectively. In reducing conditions, the molecular weight was observed at 60.8 kDa for 2D IgG heavy chain and 26.3 kDa for 2D IgG light chain, and at 62.0 kDa for 4G IgG heavy chain and 26.8 kDa for 4G IgG light chain. In addition, the concentration (amino acid concentration) of the expressed IgG heavy chain was measured to be approximately 2-fold the concentration of the expressed IgG light chain, and thus, the heavy chain having about 2-fold the amino acid sequence of the light chain is present together with the light chain at a ratio of 1:1. That is, two heavy-chain molecules and two light-chain molecules form a complex, constituting IgG.

### <2-5> Verification of PICP binding affinity of converted IgG _ indirect ELISA

The antigen binding affinity and biding specificity of IgG including 2D and 4G scFV variable regions produced in the previous examples were investigated using indirect ELISA.

PICP and PIIICP antigens prepared in <Example 1> were immobilized at a concentration of 2 ug/ml on microtiter plates at room temperature for 1 hour, and blocked with 1X PBST containing 2% skim milk at room temperature for 1 hour. 2D IgG and 4G IgG were serially diluted within a range of 15.625-1000 ng/ml, and added at 100 µl per well to the blocked plates, followed by incubation at room temperature for 1 hour. Human IgG-HRP antibodies (Millipore, 1:2,000) as secondary antibodies were added at 10 µl per well, followed by incubation at room temperature for 1 hour. The incubated microtiter plates were washed three or four times with 1X PBST, and then TMB solution was added at 50 µl per well to conduct a color development reaction at room temperature for 5-10 minutes. The color development reaction was stopped using H₂SO₄ (1 M), and then the absorbance was read at 450 nm using ELISA reader.

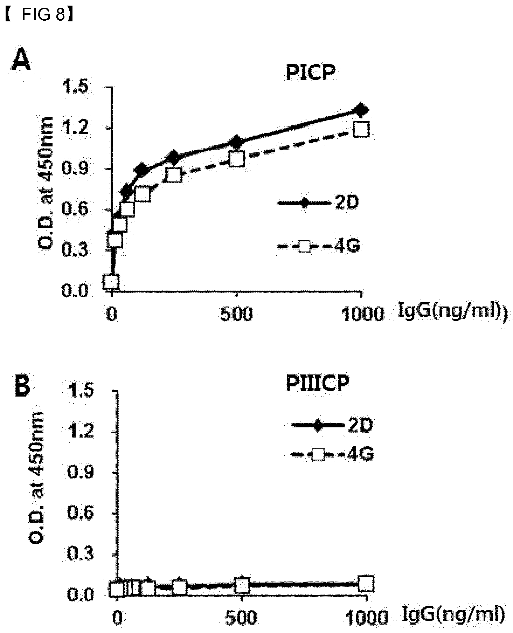

As can be confirmed in FIG. 8, both 2D IgG and 4G IgG were observed to sensitively bind to PICP antigens at low concentrations (FIG. 8A). For IgG, the IgG containing the variable regions of clone 2D was showed to have higher antigen binding affinity to PICP than the IgG containing the variable regions of clone 4G. Meanwhile, all the IgG antibodies containing the variable regions of clones 2D and 4G never did not bind to PIIICP, and thus showed very excellent binding specificity to PICP (FIG. 8B).

### <2-6> Verification of PICP binding affinity of converted IgG _ SPR

The quantitative binding affinity between the purified antibody proteins (2D IgG and 4G IgG) and the antigen (PICP) was measured using Biacore2000 SPRC (surface Plasmon resonance) (GE healthcare, USA) biosensor. After the PICP antigens were immobilized onto a sensor chip (CM5, GE healthcare , USA), antibody proteins (6.25-100 nM), which were serially diluted with HES buffer solution (10 mM HEPES, pH7.4 150 mM NaCl, 3 mM EDTA, 0.005% surfactant P20), were allowed to flow at a rate of 30 µl/min for 3 minutes, and 1 M NaCl/20mM NaOH was allowed to flow at a rate of 30 µl/min for 3 minutes, thereby inducing the dissociation of the proteins bound to the antigens. Specific experiment conditions are as follows.
Immobilized Antigen : PICP
Immobilized level : 185 RU
Antibody: 2D IgG, 4G IgG,
Running buffer: HBS- EP buffer
Regeneration : 2 M NaCl, 20 mM NaOH (flow 30 µl I min 1 min)
Binding concentration: 2D, 4G = 100 nM→50 nM→ 25 nM→12.5 nM→ 6.25 nM

**[Table 4]**

| PICP | kₐ(M⁻¹S⁻¹) | k_{d}(S⁻¹) | K_{D}(M) |
|---|---|---|---|
| 2D | 2.37X10⁵ | 2.76X10⁻³ | 1.16X10⁻⁸ |
| 4G | 1.9X10⁵ | 3.19X10⁻³ | 1.68X10⁻⁸ |

Table 4 shows kinetic rate constants and equilibrium dissociation constants of 2D IgG and 4G IgG, which were measured using Biacore 2000 SPR. The affinity was obtained from kinetic rate constants (ka and kd) and equilibrium dissociation constants (KD) using BIA evaluation ver. 3.2 software.

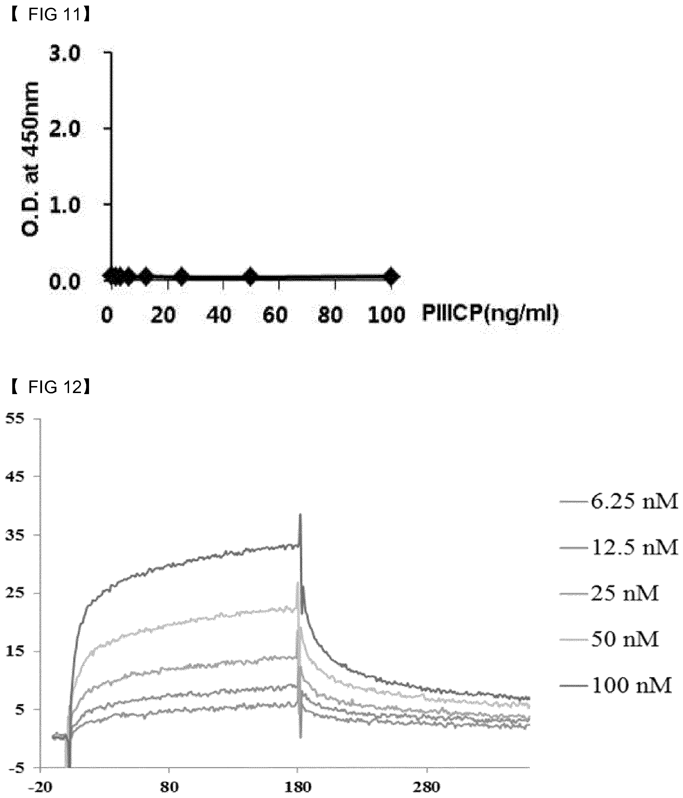

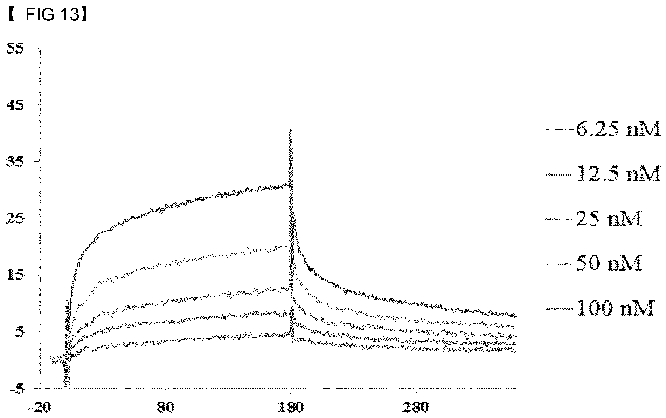

FIGS. 12 and 13 show the SPR graph results of 2D IgG and 4G IgG. The results confirmed that 2D IgG and 4G IgG had high PICP-specific binding affinity.

### <Example 3>

### Yeast Fab library screening

### <3-1> Yeast Fab selection

Yeast clones expressing Fab specifically binding to the recombinant PICP antigen proteins prepared in <Example 1> were screened by screening of yeast Fab library expressing human Fab.

A method of selecting clones specifically binding to PICP proteins from the yeast Fab library are as follows: Antigen protein PICP and similar protein PIIICP were prepared with purity of 99% or higher by the method of the present invention. The PICP proteins were conjugated to biotin using a kit (EZ-LINKTMSulfo-NHS-LC-Biotinylation kit, Pierce Inc., USA). Thereafter, a procedure of screening the constructed libraries using MACS and FACS and analyzing the induced pool was repeated. Through this, the expression level of antibody Fab and the binding affinity of biotinylated PICP can be analyzed.

The biotinylated PICP (500 nM) was conjugated to Fab library expressed on the yeast cell surface at 25□ for 1 hour. The Fab library incubated with PICP was conjugated with streptavidin-microbead (Miltenyi Biotec Inc., Germany) at 4□ for 10 minutes, and then clones conjugated with the biotinylated PICP were selected using magnetic-activated cell sorting (MACS). Then, PICP was bound to the Fab library expressed on the yeast cell surface at 25□ for 1 hour, and thereafter, PE-conjugated streptavidin (streptavidin-R-phycoerythrin conjugate (SA-PE), Invitrogen) and Alexa 488-conjugated anti-IgG antibody (goat Alexa 488-conjugated anti-Fc antibody, SIGMA-ALDRICH Co., USA) were bound thereto at 4□ for 20 minutes, thereby selecting clones having high expression levels of Fab and high binding affinity with biotinylated PICP were selected using fluorescence-activated cell sorting (FACS). Thereafter, FACS procedure was twice repeated using biotinylated PICP with concentrations of 250 nM and 100 nM. In the secondary and tertiary FACS procedures, PICP conjugated without biotin, as a competitor protein of biotinylated PICP, was allowed to compete at a 10-fold higher concentration for execution of secondary FACS and a 20-fold higher concentration for execution of tertiary FACS, thereby selecting individual clones binding to PICP.

Clone 1 and clone 2 were finally selected as the clones specifically binding to PICP, and DNA was obtained from the yeast cells for each, followed by sequencing, thereby confirming nucleotide sequences and amino acid sequences corresponding to Fab.

### <3-2> Conversion of Fab into IgG

In order to convert clone 1 Fab and clone 2 Fab selected in <example 3-1> into IgG format and express the Fab fragments in animal cells, nucleotide sequences encoding Fab VH and VL were confirmed from DNA isolated from the clones, followed by cloning, and the clones were inserted into pcDNA3.4 vectors containing mouse IgG2a heavy and light chains, respectively.

Specifically, the VH portion was amplified by PCR using primers (forward 5'-AATGTACACTCCGAAGTGCAATTGGTGGAGTCTG-3', SEQ ID NO: 103; and reverse 5'-GACCGATGGGGCTGTTGTTTTGGCGGAAGAGACGGTAACC-3', SEQ ID NO: 104). Heavy chain fragments were amplified from vectors containing CHI, CH2, and CH3 of mouse IgG 2a by conducting PCR using primers (forward 5'-GCCAAAACAACAGCCCCATCGGTC-3', SEQ ID NO: 105; and reverse 5'-ATATCCAAGCTTCTACTATTTACCCGGAGTCCGGGA G-3', SEQ ID NO: 106) Thereafter, overlap extension PCR was performed using the primers of SEQ ID NO: 103 and SEQ ID NO: 106 to construct chimeric IgG 2α heavy chain DNA, which was then introduced into pcDNA3.4, which is a vector used in the production of IgG, by treatment with proper restriction enzymes.

For the VL portion, fragments thereof were amplified by PCR using primers (forward 5'-CTCGGTCATAATGTCCAGAGGAGATATTCAGATGACACAGTCTCC-3', SEQ ID NO: 107; and reverse 5'-TTCGTACGCTTAATCTCCACCTTC-3', SEQ ID NO: 108). Light chain fragments were amplified from vectors including mouse kappa light chain using primers (forward 5'-CTTTTACATTCGGCCAGGGAACGAAGGTGGAGATTAAGCGT-3', SEQ ID NO: 109; reverse 5'-ATCCAAGCTTCTACTAACACTCATTCCTGTTGAAG-3', SEQ ID NO: 110). Thereafter, overlap extension PCR was performed using the primers of SEQ ID NO: 107 and SEQ ID NO: 110 to construct chimeric IgG 2α light chain DNA, which was then introduced into pcDNA3.4, which is a vector used in the production of IgG, by treatment with proper restriction enzymes

### <3-3> Verification of PICP binding affinity of converted IgG

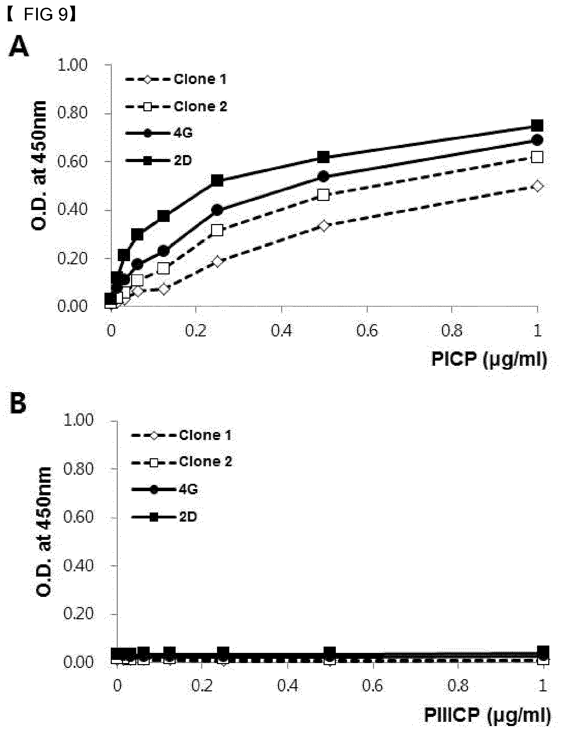

The binding specificity to PICP by IgG fragment having VH and VL of clones 1 and 2 (referred to as clone 1 IgG and clone 2 IgG) was investigated by sandwich ELISA (FIG. 9). PICP and PIIICP were used as antigens of sandwich ELISA, and ELISA was performed with 2D IgG and 4G IgG produced in the previous example to compare the binding affinity and binding specificity of newly produced IgG.

2D, 4G, clone 1 , clone 2 IgG antibodies as capture antibodies were coated on the microtiter plates, and PICP and PIIICP as antigens were added to each well (100 µl/well) at different concentrations, followed by incubation for 1 hour. After washing with 1X PBST (0.05 Tween 20), detection antibodies (polyclonal PICP antibodies) were diluted with 1X PBST, and added in 100 µl for each, followed by incubation for 1 hour. After washing with 1X PBST, rabbit-HRP (Millipore) antibodies were diluted with 1X PBST, and added in 100 µl for each, followed by incubation for 1 hour. After washing with 1X PBST, TMB solution was added to each well (50 µl/well) to investigate a color development reaction, and then the reaction was stopped by addition of 50 µl of 2N stop solution (H2S04). The absorbance was read at a wavelength of 450 nm using ELISA reader (reference 620 nm) .

As can be confirmed from FIG. 9, clone 1 IgG and clone 2 IgG also sensitively responded to PIPC, and the binding affinity levels thereof to PICP was somewhat lower compared with those of the previously selected 4G IgG and 2D IgG (FIG. 9A) . Of the IgG antibodies, the antigen binding affinity of 2D IgG was very excellent. In addition, both clone 1 IgG and clone 2 IgG never did not bind to PIIICP, and thus showed very excellent binding specificity to PICP (FIG. 9B).

### <Example 4>

### ELISA efficiency using IgG

ELISA efficiency was investigated by additionally performing sandwich ELISA of 2D IgG, which has the highest antigen binding affinity among the selected IgG antibodies.

2D IgG was immobilized at a concentration of 16 ug/ml on microtiter (96 well) plates at room temperature for 2 hours, and blocking was conducted at room temperature for 30 minutes by addition of 1X PBST containing 1% BSA in 300 µl per well. After washing three times with 200 µl of 1X PBST, stabilization was conducted for 10 minutes by addition of 2% sucrose (200 µl/well). After 10 minutes, the sucrose solution was drained, followed by drying at room temperature for 10 minutes. The PICP antigens or the samples to be measured for concentration were diluted with 1X PBST for different concentrations, and added in 100 µl per well, followed by incubation at room temperature for 30 minutes. The reaction solution was removed, and rabbit anti-PICP antibodies as detection antibodies were added at a concentration 2 ug/ml (100 µl/well), followed by incubation for 30 minutes. Goat anti-rabbit IgG-HRP (Milipore) antibodies as secondary antibodies were added (100 µl/well), followed by incubation at room temperature for 1 hour. After washing four times with 1X PBST, TMB solution was added (50 µl/well), followed by a color development reaction at room temperature for 5-15 minutes. The color development reaction was stopped by addition of H₂SO₄ (2N) in 50 µl per well, and the absorbance was read at 450 nm using ELISA reader. The measured absorbance was converted into the concentration by 4-parameter method to investigate the concentrations of the standard solution and samples.

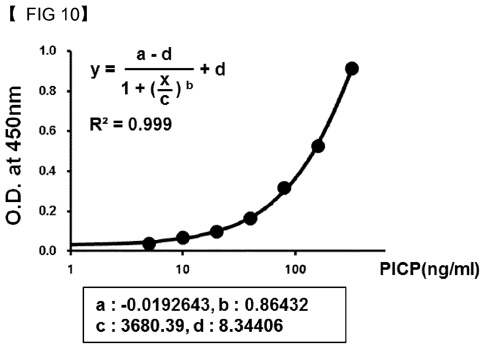

FIG. 10 depicts a standard curve showing absorbance with the concentration of PICP antigen. The absorbance measurement values used for the standard curve are shown in Table 5, and PICP related data converted by the standard curve are shown in Table 6. The limit of detection (IOD) in ELISA using 2D IgG is verified to be 1 ng/ml. It was confirmed that for accuracy, the coefficient of variation (CV) measured according to the antigen concentration was 20% or less, and the recovery rate was in the range of 80-120%.

As shown in FIG. 11, 2D IgG never did not bind to PIIICP, and thus showed no cross-reactivity to PIIICP.

**[Table 5]**

| **PICP ELISA absorbance** | |
|---|---|
| **2D** | |
| **PICP (ng/ml)** | **Abs. 450nm** |
| **320** | **0.914** |
| **160** | **0.526** |
| **80** | **0.317** |
| **40** | **0.165** |
| **20** | **0.098** |
| **10** | **0.065** |
| **5** | **0.031** |
| **0** | **0.029** |

**[Table 6]**

| **PICP ELISA PERFORMANCE EVALUATION** | | | | |
|---|---|---|---|---|
| **Sample (ng/ml))** | **Conc. Ave. (ng/ml)** | **Stdev (ng/ml)** | **CV(%) (<20%)** | **Recovery(%) (80-120%)** |
| **100** | **94.64** | **2.47** | **2.61** | **94.64** |
| **50** | **46.84** | **3.78** | **8.06** | **93.67** |
| **25** | **22.82** | **2.99** | **13.09** | **91.27** |
| **12.5** | **11.52** | **1.01** | **8.75** | **92.19** |
| **6.25** | **6.04** | **0.83** | **13.76** | **96.65** |
| **3.125** | **3.08** | **0.47** | **15.40** | **98.53** |
| **1.5625** | **1.58** | **0.18** | **11.36** | **101.20** |

### Industrial applicability

As described above, the protein complex PICP can be easily produced at high purity and high yield, and antibodies specific thereto can be produced by using the present invention. The antibodies and methods according to the present invention can be favorably used in the development of PICP detection kits or diagnostic reagents for various purposes, for example, for a medical use or cosmetic efficacy measurement, to confirm collagen synthesis or collagen-related metabolism by sensing PICP.

## Claims

1. An antibody or fragment thereof specifically binding to procollagen type I C-terminal propeptide (PICP), the antibody or fragment thereof comprising:
a heavy chain variable domain (VH) comprising a heavy chain complementary determining region 1 (CDR1) containing an amino acid sequence selected from the group consisting of SEQ ID NO: 1, SEQ ID NO: 13, SEQ ID NO: 25, and SEQ ID NO: 37, a heavy chain complementary determining region 2 (CDR2) containing an amino acid sequence selected from the group consisting of SEQ ID NO: 3, SEQ ID NO: 15, SEQ ID NO: 27, and SEQ ID NO: 39, and a heavy chain complementary determining region 3 (CDR3) containing an amino acid sequence selected from the group consisting of SEQ ID NO: 5, SEQ ID NO: 17, SEQ ID NO: 29, and SEQ ID NO: 41; and
a light chain variable domain (VH) comprising a light chain complementary determining region 1 (CDR1) containing an amino acid sequence selected from the group consisting of SEQ ID NO: 7, SEQ ID NO: 19, SEQ ID NO: 31, and SEQ ID NO: 43, a light chain complementary determining region 2 (CDR2) containing an amino acid sequence selected from the group consisting of SEQ ID NO: 9, SEQ ID NO: 21, SEQ ID NO: 33, and SEQ ID NO: 45, and a light chain complementary determining region 3 (CDR3) containing an amino acid sequence selected from the group consisting of SEQ ID NO: 11, SEQ ID NO: 23, SEQ ID NO: 35, and SEQ ID NO: 47.

2. The antibody or fragment thereof of claim 1, wherein the antibody or fragment thereof comprises a heavy chain variable domain and a light chain variable domain selected from the group consisting of:
a heavy chain variable domain comprising heavy chain complementary determining region 1 containing the amino acid sequence represented by SEQ ID NO: 1, heavy chain complementary determining region 2 containing the amino acid sequence represented by SEQ ID NO: 3, and heavy chain complementary determining region 3 containing the amino acid sequence represented by SEQ ID NO: 5; and a light chain variable domain including light chain complementary determining region 1 containing the amino acid sequence represented by SEQ ID NO: 7, light chain complementary determining region 2 containing the amino acid sequence represented by SEQ ID NO: 9, and light chain complementary determining region 3 containing the amino acid sequence represented by SEQ ID NO: 11;
a heavy chain variable domain comprising heavy chain complementary determining region 1 containing the amino acid sequence represented by SEQ ID NO: 13, heavy chain complementary determining region 2 containing the amino acid sequence represented by SEQ ID NO: 15, and heavy chain complementary determining region 3 containing the amino acid sequence represented by SEQ ID NO: 17; and a light chain variable domain comprising light chain complementary determining region 1 containing the amino acid sequence represented by SEQ ID NO: 19, light chain complementary determining region 2 containing the amino acid sequence represented by SEQ ID NO: 21, and light chain complementary determining region 3 containing the amino acid sequence represented by SEQ ID NO: 23;
a heavy chain variable domain comprising heavy chain complementary determining region 1 containing the amino acid sequence represented by SEQ ID NO: 25, heavy chain complementary determining region 2 containing the amino acid sequence represented by SEQ ID NO: 27, and heavy chain complementary determining region 3 containing the amino acid sequence represented by SEQ ID NO: 29; and a light chain variable domain comprising light chain complementary determining region 1 containing the amino acid sequence represented by SEQ ID NO: 31, light chain complementary determining region 2 containing the amino acid sequence represented by SEQ ID NO: 33, and light chain complementary determining region 3 containing the amino acid sequence represented by SEQ ID NO: 35; and
a heavy chain variable domain comprising heavy chain complementary determining region 1 containing the amino acid sequence represented by SEQ ID NO: 37, heavy chain complementary determining region 2 containing the amino acid sequence represented by SEQ ID NO: 39, and heavy chain complementary determining region 3 containing the amino acid sequence represented by SEQ ID NO: 41; and a light chain variable domain comprising light chain complementary determining region 1 containing the amino acid sequence represented by SEQ ID NO: 43, light chain complementary determining region 2 containing the amino acid sequence represented by SEQ ID NO: 45, and light chain complementary determining region 3 containing the amino acid sequence represented by SEQ ID NO: 47.

3. The antibody or fragment thereof of claim 1, wherein the heavy chain variable domain contains an amino acid sequence selected from the group consisting of SEQ ID NO: 49, SEQ ID NO: 53, SEQ ID NO: 57, and SEQ ID NO: 61, and the light chain variable domain contains an amino acid sequence selected from the group consisting of SEQ ID NO: 51, SEQ ID NO: 55, SEQ ID NO: 59, and SEQ ID NO: 63.

4. The antibody or fragment thereof of claim 1, wherein the antibody is composed of a heavy chain containing an amino acid sequence of SEQ ID NO: 69, SEQ ID NO: 73, SEQ ID NO: 77, and SEQ ID NO: 81; and a light chain containing an amino acid sequence of SEQ ID NO: 71, SEQ ID NO: 75, SEQ ID NO: 79, and SEQ ID NO: 83.

5. The antibody or fragment thereof of claim 1, wherein the antibody is selected from the group consisting of IgG, IgA, IgM, IgE, and IgD, and the fragment is selected from the group consisting of diabody, Fab, Fab', F(ab)2, F(ab')2, Fv, and scFv.

6. The antibody or fragment thereof of claim 5, wherein the scFv contains an amino acid sequence represented by SEQ ID NO: 65 or SEQ ID NO: 67.

7. The antibody or fragment thereof of claim 5, wherein the Fab contains amino acid sequences represented by SEQ ID NO: 57 and SEQ ID NO: 59, or SEQ ID NO: 61 and SEQ ID NO: 63.

8. An enzyme-linked immunosorbent assay (ELISA) kit comprising the antibody or fragment thereof of any one of claims 1 to 7.

9. A composition for diagnosing a metabolic bone disease, the composition comprising the antibody or fragment thereof of any one of claims 1 to 7.

10. The composition of claim 9, wherein the metabolic bone disease is selected from the group consisting of osteoporosis, Paget's disease, osteodystrophy, osteogenesis imperfecta, bone cancer, metastatic bone diseases, osteomalacia, osteopenia, bone atrophy, fibrous dysplasia, hypercalcemia, osteolysis, osteoarthritis, periodontal diseases, and rheumatoid arthritis.

11. A PICP-specific detection method comprising:
(1) preparing a sample;
(2) contacting the antibody or fragment of any one of claims 1 to 7 with the sample; and
(3) detecting the antibody or fragment thereof.

12. A polynucleotide encoding the antibody or fragment thereof of any one of claims 1 to 7.

13. The polynucleotide of claim 12, wherein the polynucleotide contains a nucleotide sequence represented by SEQ ID NO: 66 or SEQ ID NO: 68.

14. The polynucleotide of claim 12, wherein the polynucleotide contains a nucleotide sequence selected from the group consisting of SEQ ID NO: 70, SEQ ID NO: 72, SEQ ID NO: 74, SEQ ID NO: 76, SEQ ID NO: 78, SEQ ID NO: 80, SEQ ID NO: 82, and SEQ ID NO: 84.

15. A recombinant expression vector comprising the polynucleotide of claim 12.

16. A cell transfected with the recombinant expression vector of claim 15.

17. A method for preparing an antibody or fragment thereof specifically binding to PICP, the method comprising:
(a) transfecting a host cell with the recombinant expression vector of claim 15;
(b) culturing the transfected host cell to prepare an antibody or fragment thereof; and
(c) harvesting the antibody or fragment thereof prepared in the host cell.

18. A method for preparing a recombinant PICP protein, the method comprising:
(i) constructing recombinant expression vectors comprising polynucleotides encoding PICP α1 chain and PICP α2 chain, respectively, to which a signal peptide stimulating extracellular secretion and a label protein are conjugated;
(ii) co-transfecting a cell with a mixture of the recombinant expression vector comprising the polynucleotide encoding PICP α1 chain and the recombinant expression vector comprising the polynucleotide encoding PICP α2 chain;
(iii) culturing the co-transfected cell; and
(iv) obtaining a PICP protein produced in the cell.

19. The method of claim 18, wherein the polynucleotide encoding PICP α1 chain and the polynucleotide encoding PICP α2 chain contain the nucleotide sequences represented by SEQ ID NO: 86 and SEQ ID NO: 88, respectively.

20. Use of the antibody or fragment thereof of any one of claims 1 to 7 for preparing an agent for diagnosis of a metabolic bone disease.

21. A method for diagnosing a metabolic bone disease in a subject, the method comprising:
(a) obtaining a biological sample from a subject;
(b) determining the level of PICP protein in the biological sample using the antibody or fragment of any one of claims 1 to 7; and
(c) comparing the determined level of PICP protein with the level of PICP protein in a normal subject.


<110>	Medicinal Bioconvergence Research Center

<120>	Methods for producing recombinant PICP proteins and antibodies
	specifically binding thereto

<130>	OP16-0117

<150>	KR 10-2015-0189684
<151>	2015-12-30

<160>	112

<170>	KopatentIn 2.0

<210>	1
<211>	5
<212>	PRT
<213>	Artificial Sequence

<220>
<223>	2D VH CDR1

<400>	1
Ser Tyr Tyr Met Ser
  1               5

<210>	2
<211>	15
<212>	DNA
<213>	Artificial Sequence

<220>
<223>	2D VH CDR1

<400>	2
agttattata tgagc                                                        15

<210>	3
<211>	17
<212>	PRT
<213>	Artificial Sequence

<220>
<223>	2D VH CDR2

<400>	3
Gly Ile Ser Pro Gly Ser Gly Ser Lys Tyr Tyr Ala Asp Ser Val Lys
  1               5                  10                  15

Gly

<210>	4
<211>	51
<212>	DNA
<213>	Artificial Sequence

<220>
<223>	2D VH CDR2

<400>	4

gggatctctc ctggtagtgg tagtaaatat tacgctgatt ctgtaaaagg t 51

<210> 5
<211> 18
<212> PRT
<213> Artificial Sequence

<220>
<223> 2D VH CDR3

<400> 5
Ala Thr Val Pro Cys Lys Gly Pro Ala Cys Tyr Ser Ala Tyr Gly Met
1               5                   10                  15

Asp Val

<210> 6
<211> 54
<212> DNA
<213> Artificial Sequence

<220>
<223> 2D VH CDR3

<400> 6
gctactgttc cgtgtaaggg gcctgcgtgt tattctgctt atggtatgga cgtc 54

<210> 7
<211> 11
<212> PRT
<213> Artificial Sequence

<220>
<223> 2D VL CDR1

<400> 7
Ser Ser Ser Asn Ile Gly Asn Asn Ala Val Asn
1               5                   10

<210> 8
<211> 33
<212> DNA
<213> Artificial Sequence

<220>
<223> 2D VL CDR1

<400> 8
tcttcatcta atattggcaa taatgctgtc aac 33

<210> 9
<211> 7
<212> PRT
<213> Artificial Sequence

<220>
<223> 2D VL CDR2

<400>    9
Ser Asn Ser His Arg Pro Ser
  1               5


<210>    10
<211>    21
<212>    DNA
<213>    Artificial Sequence

<220>
<223>    2D VL CDR2


<400>    10
tctaatagtc atcggccaag c                                                    21


<210>    11
<211>    9
<212>    PRT
<213>    Artificial Sequence

<220>
<223>    2D VL CDR3


<400>    11
Gly Ser Trp Asp Ala Ser Leu Asn Gly
  1               5


<210>    12
<211>    27
<212>    DNA
<213>    Artificial Sequence

<220>
<223>    2D VL CDR3


<400>    12
ggttcttggg atgctagcct gaatggt                                              27


<210>    13
<211>    5
<212>    PRT
<213>    Artificial Sequence

<220>
<223>    4G VH CDR1


<400>    13
Asn Tyr Tyr Met Ser
  1               5


<210>    14
<211>    15
<212>    DNA
<213>    Artificial Sequence

<220>
<223>    4G VH CDR1

<400>    14
aattattata tgagc                                                        15


<210>    15
<211>    17
<212>    PRT
<213>    Artificial Sequence

<220>
<223>    4G VH CDR2


<400>    15
Leu Ile Ser Ser Gly Asn Gly Asn Lys Tyr Tyr Ala Asp Ser Val Lys
1               5                   10                  15

Gly



<210>    16
<211>    51
<212>    DNA
<213>    Artificial Sequence

<220>
<223>    4G VH CDR2


<400>    16
ttgatctctt ctggtaatgg taataaatat tacgctgatt ctgtaaaagg t                51


<210>    17
<211>    18
<212>    PRT
<213>    Artificial Sequence

<220>
<223>    4G VH CDR3


<400>    17
Gly Ser Arg Arg Cys Ser Gly Ala Pro Cys Tyr Ser Ala Tyr Gly Met
1               5                   10                  15

Asp Val



<210>    18
<211>    54
<212>    DNA
<213>    Artificial Sequence

<220>
<223>    4G VH CDR3


<400>    18
ggtagtcgtc ggtgtagtgg tgctccttgt tattctgctt atggtatgga cgtc             54


<210>    19
<211>    11
<212>    PRT

<213>    Artificial Sequence

<220>
<223>    4G VL CDR1

<400>    19
Ser Ser Ser Asn Ile Gly Asn Asn Ser Val Thr
1               5                   10

<210>    20
<211>    33
<212>    DNA
<213>    Artificial Sequence

<220>
<223>    4G VL CDR1

<400>    20
tcttcatcta atattggcaa taattctgtc acc                                    33

<210>    21
<211>    7
<212>    PRT
<213>    Artificial Sequence

<220>
<223>    4G VL CDR2

<400>    21
Ala Asn Ser Gln Arg Pro Ser
1               5

<210>    22
<211>    21
<212>    DNA
<213>    Artificial Sequence

<220>
<223>    4G VL CDR2

<400>    22
gctaatagtc agcggccaag c                                                 21

<210>    23
<211>    9
<212>    PRT
<213>    Artificial Sequence

<220>
<223>    4G VL CDR3

<400>    23
Gly Ala Trp Asp Asp Ser Leu Asn Ala
1               5

<210>    24
<211>    27
<212>    DNA

<213>    Artificial Sequence

<220>
<223>    4G VL CDR3


<400>    24
ggtgcttggg atgatagcct gaatgct                                          27


<210>    25
<211>    5
<212>    PRT
<213>    Artificial Sequence

<220>
<223>    clone 1 VH CDR1


<400>    25
Tyr Tyr Trp Met Ser
  1               5


<210>    26
<211>    15
<212>    DNA
<213>    Artificial Sequence

<220>
<223>    clone 1 VH CDR1


<400>    26
tattactgga tgagc                                                       15


<210>    27
<211>    17
<212>    PRT
<213>    Artificial Sequence

<220>
<223>    clone 1 VH CDR2


<400>    27
Thr Ile Ser Tyr Gly Gly Gly Gly Lys Tyr Tyr Ala Asp Ser Val Lys
  1               5                   10                  15

Gly



<210>    28
<211>    51
<212>    DNA
<213>    Artificial Sequence

<220>
<223>    clone 1 VH CDR2


<400>    28
accatttcgt atggtggagg tggtaaatac tacgcagatt ctgtcaaggg c               51


<210>    29

<211>	12
<212>	PRT
<213>	Artificial Sequence

<220>
<223>	clone 1 VH CDR3

<400>	29
Ser Tyr Gly Ala Ile Pro Pro Asn Thr Ile Asp Tyr
1               5                   10

<210>	30
<211>	36
<212>	DNA
<213>	Artificial Sequence

<220>
<223>	clone 1 VH CDR3

<400>	30
tcgtatggtg cgattccgcc taatactatt gactac                                36

<210>	31
<211>	11
<212>	PRT
<213>	Artificial Sequence

<220>
<223>	clone 1 VL CDR1

<400>	31
Arg Ala Ser Gln Gly Ile Ser Ser Asp Leu Ala
1               5                   10

<210>	32
<211>	33
<212>	DNA
<213>	Artificial Sequence

<220>
<223>	clone 1 VL CDR1

<400>	32
agggcctctc aaggcatctc ctctgatttg gca                                   33

<210>	33
<211>	7
<212>	PRT
<213>	Artificial Sequence

<220>
<223>	clone 1 VL CDR2

<400>	33
Ala Ala Ser Ser Leu Gln Ser
1               5

<210>	34

<211>	21
<212>	DNA
<213>	Artificial Sequence

<220>
<223>	clone 1 VL CDR2


<400>	34
gctgcttctt ctttgcaatc t	21


<210>	35
<211>	9
<212>	PRT
<213>	Artificial Sequence

<220>
<223>	clone 1 VL CDR3


<400>	35
Gln Gln Pro Tyr Ser Thr Pro Ser Thr
1               5


<210>	36
<211>	27
<212>	DNA
<213>	Artificial Sequence

<220>
<223>	clone 1 VL CDR3


<400>	36
caacaacctt attctactcc ttctaca	27


<210>	37
<211>	5
<212>	PRT
<213>	Artificial Sequence

<220>
<223>	clone 2 VH CDR1


<400>	37
Ser Tyr Trp Met Ser
1               5


<210>	38
<211>	15
<212>	DNA
<213>	Artificial Sequence

<220>
<223>	clone 2 VH CDR1


<400>	38
tcttactgga tgagc	15


<210>	39
<211>	17

<212>	PRT
<213>	Artificial Sequence

<220>
<223>	clone 2 VH CDR2


<400>	39
Val Ile Ser Tyr Asp Gly Gly Asn Lys Tyr Tyr Ala Asp Ser Val Lys
1               5                   10                  15

Gly




<210>	40
<211>	51
<212>	DNA
<213>	Artificial Sequence

<220>
<223>	clone 2 VH CDR2


<400>	40
gtcatttcgt atgatggagg taataaatac tacgcagatt ctgtcaaggg c	51


<210>	41
<211>	13
<212>	PRT
<213>	Artificial Sequence

<220>
<223>	clone 2 VH CDR3


<400>	41
Trp Ala Asn Asp His Asn Ser Asp Leu Asn Phe Asp Tyr
1               5                   10


<210>	42
<211>	39
<212>	DNA
<213>	Artificial Sequence

<220>
<223>	clone 2 VH CDR3


<400>	42
tgggctaatg atcataattc tgatcttaat tttgactac	39


<210>	43
<211>	11
<212>	PRT
<213>	Artificial Sequence

<220>
<223>	clone 2 VL CDR1


<400>	43
Arg Ala Ser Gln Gly Ile Ser Thr Asp Leu Gly
1               5                   10

<210>	44
<211>	33
<212>	DNA
<213>	Artificial Sequence

<220>
<223>	clone 2 VL CDR1


<400>	44
agggcctctc aaggcatctc cactgatttg gga	33


<210>	45
<211>	7
<212>	PRT
<213>	Artificial Sequence

<220>
<223>	clone 2 VL CDR2


<400>	45
Ala Ala Ser Ser Leu Glu Ser
1               5


<210>	46
<211>	21
<212>	DNA
<213>	Artificial Sequence

<220>
<223>	clone 2 VL CDR2


<400>	46
gctgcttctt ctttggaatc t	21


<210>	47
<211>	9
<212>	PRT
<213>	Artificial Sequence

<220>
<223>	clone 2 VL CDR3


<400>	47
Gln Gln His Asp Ser Tyr Pro Thr Thr
1               5


<210>	48
<211>	27
<212>	DNA
<213>	Artificial Sequence

<220>
<223>	clone 2 VL CDR3


<400>	48
caacaacatg attcttatcc tactaca	27

<210>    49
<211>    127
<212>    PRT
<213>    Artificial Sequence

<220>
<223>    2D VH


<400>    49
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Tyr Met Ser Trp Val Arg Gln Ala Pro Gly Lys Gly Leu Glu Trp Val
        35                  40                  45

Ser Gly Ile Ser Pro Gly Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ala Thr Val Pro Cys Lys Gly Pro Ala Cys Tyr Ser Ala Tyr
            100                 105                 110

Gly Met Asp Val Trp Gly Gln Gly Thr Leu Val Thr Val Ser Ser
        115                 120                 125




<210>    50
<211>    381
<212>    DNA
<213>    Artificial Sequence

<220>
<223>    2D VH


<400>    50
gaggtgcagc tgttggagtc tgggggaggc ttggtacagc ctggggggtc cctgagactc      60

tcctgtgcag cctctggatt cacctttagc agttattata tgagctgggt ccgccaggct     120

ccagggaagg ggctggagtg ggtctcaggg atctctcctg gtagtggtag taaatattac     180

gctgattctg taaaaggtcg gttcaccatc tccagagaca attccaagaa cacgctgtat     240

ctgcaaatga acagcctgag agccgaggac acggccgtgt attactgtgc gagagctact     300

gttccgtgta aggggcctgc gtgttattct gcttatggta tggacgtctg gggccagggt     360

acactggtca ccgtgagctc a                                               381

<210>    51
<211>    109
<212>    PRT
<213>    Artificial Sequence

<220>

<223>    2D VL

<400>    51
Gln Ser Val Leu Thr Gln Pro Pro Ser Ala Pro Gly Thr Pro Gly Gln
1               5                   10                  15

Arg Val Thr Ile Ser Cys Ser Gly Ser Ser Ser Asn Ile Gly Asn Asn
            20                  25                  30

Ala Val Asn Trp Tyr Gln Gln Leu Pro Gly Thr Ala Pro Lys Leu Leu
        35                  40                  45

Ile Tyr Ser Asn Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val
            100                 105

<210>    52
<211>    327
<212>    DNA
<213>    Artificial Sequence

<220>
<223>    2D VL

<400>    52
cagtctgtgc tgactcagcc accctcagcg cctgggaccc ccgggcagag ggtcaccatc    60

tcttgtagtg gctcttcatc taatattggc aataatgctg tcaactggta ccagcagctc    120

ccaggaacgg cccccaaact cctcatctat tctaatagtc atcggccaag cggggtccct    180

gaccgattct ctggctccaa gtctggcacc tcagcctccc tggccatcag tgggctccgg    240

tccgaggatg aggctgatta ttactgtggt tcttgggatg ctagcctgaa tggttatgtc    300

ttcggcggag gcaccaagct gacggtc                                        327

<210>    53
<211>    127
<212>    PRT
<213>    Artificial Sequence

<220>
<223>    4G VH

<400>    53
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Leu Ile Ser Ser Gly Asn Gly Asn Lys Tyr Tyr Ala Asp Ser Val
50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Arg Arg Cys Ser Gly Ala Pro Cys Tyr Ser Ala Tyr
            100                 105                 110

Gly Met Asp Val Trp Gly Gln Gly Thr Leu Val Thr Val Ser Ser
        115                 120                 125

<210>   54
<211>   381
<212>   DNA
<213>   Artificial Sequence

<220>
<223>   4G VH

<400>   54
gaggtgcagc tgttggagtc tggggggggc ttggtacagc ctggggggtc cctgagactc      60

tcctgtgcag cctctggatt cacctttagc aattattata tgagctgggt ccgccaggct      120

ccagggaagg ggctggagtg ggtctcattg atctcttctg gtaatggtaa taaatattac      180

gctgattctg taaaaggtcg gttcaccatc tccagagaca attccaagaa cacgctgtat      240

ctgcaaatga acagcctgag agccgaggac acggccgtgt attactgtgc gagaggtagt      300

cgtcggtgta gtggtgctcc ttgttattct gcttatggta tggacgtctg gggccagggt      360

acactggtca ccgtgagctc a                                                381

<210>   55
<211>   109
<212>   PRT
<213>   Artificial Sequence

<220>
<223>   4G VL

<400>   55
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asn Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Leu
                85                  90                  95

Asn Ala Tyr Val Phe Gly Gly Gly Thr Lys Leu Thr Val
            100                 105

<210>   56
<211>   327
<212>   DNA
<213>   Artificial Sequence

<220>
<223>   4G VL

<400>   56
cagtctgtgc tgactcagcc accctcagcg tctgggaccc ccgggcagag ggtcaccatc      60

tcttgtactg gctcttcatc taatattggc aataattctg tcacctggta ccagcagctc     120

ccaggaacgg cccccaaact cctcatctat gctaatagtc agcggccaag cggggtccct     180

gaccgattct ctggctccaa gtctggcacc tcagcctccc tggccatcag tgggctccgg     240

tccgaggatg aggctgatta ttactgtggt gcttgggatg atagcctgaa tgcttatgtc     300

ttcggcggag gcaccaagct gacggtc                                         327

<210>   57
<211>   121
<212>   PRT
<213>   Artificial Sequence

<220>
<223>   clone 1 VH

<400>   57
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Tyr Tyr
            20                  25                  30

Trp Met Ser Trp Val Arg Gln Ala Pro Gly Lys Gly Leu Glu Trp Val
        35                  40                  45

Ser Thr Ile Ser Tyr Gly Gly Gly Gly Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Arg Ser Tyr Gly Ala Ile Pro Pro Asn Thr Ile Asp Tyr Trp Gly
            100                 105                 110

Gln Gly Thr Leu Val Thr Val Ser Ser
        115                 120

<210>   58
<211>   363
<212>   DNA

<213>    Artificial Sequence

<220>
<223>    clone 1 VH

<400>    58
gaagtgcaat tggtggagtc tggcggcgga ttggtgcaac caggaggatc tttgagattg    60

tcttgcgcag cctctggatt cacattctct tattactgga tgagctgggt caggcaggct    120

ccaggaaaag gcttggaatg ggtgtcaacc atttcgtatg gtggaggtgg taaatactac    180

gcagattctg tcaagggcag gttcaccatc tccagggaca actccaagaa cacattgtac    240

ttgcagatga attccttgag agctgaagac acggctgtgt attactgtgt taggtcgtat    300

ggtgcgattc cgcctaatac tattgactac tggggacagg gaacattggt taccgtctct    360

tcc                                                                  363

<210>    59
<211>    108
<212>    PRT
<213>    Artificial Sequence

<220>
<223>    clone 1 VL

<400>    59
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Asp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Tyr Ser Thr Pro Ser
                85                  90                  95

Thr Phe Gly Gln Gly Thr Lys Val Glu Ile Lys Arg
            100                 105

<210>    60
<211>    324
<212>    DNA
<213>    Artificial Sequence

<220>
<223>    clone 1 VL

<400>    60
gatattcaga tgacacagtc tccttcctct ttgtctgctt ctgtgggcga tagggttaca    60

ataacatgca gggcctctca aggcatctcc tctgatttgg catggtatca gcagaagcct    120

ggaaaggctc ctaaattgtt gatctatgct gcttcttctt tgcaatctgg agttccttct 180

agattttctg gatctggatc tggaacagac ttcacattga ccatatcctc cttgcaacct 240

gaagatttcg ctacatatta ctgccaacaa ccttattcta ctccttctac attcggccag 300

ggaacgaagg tggagattaa gcgt 324

<210> 61
<211> 122
<212> PRT
<213> Artificial Sequence

<220>
<223> clone 2 VH

<400> 61
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ser Val Ile Ser Tyr Asp Gly Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Trp Ala Asn Asp His Asn Ser Asp Leu Asn Phe Asp Tyr Trp
            100                 105                 110

Gly Gln Gly Thr Leu Val Thr Val Ser Ser
        115                 120

<210> 62
<211> 366
<212> DNA
<213> Artificial Sequence

<220>
<223> clone 2 VH

<400> 62
gaagtgcaat tggtggagtc tggcggcgga ttggtgcaac caggaggatc tttgagattg 60

tcttgcgcag cctctggatt cacattctct tcttactgga tgagctgggt caggcaggct 120

ccaggaaaag gcttggaatg ggtgtcagtc atttcgtatg atggaggtaa taaatactac 180

gcagattctg tcaagggcag gttcaccatc tccagggaca actccaagaa cacattgtac 240

ttgcagatga attccttgag agctgaagac acggctgtgt attactgtgc taagtgggct 300

aatgatcata attctgatct taattttgac tactggggac agggaacatt ggttaccgtc 360

tcttcc 366

<210>    63
<211>    108
<212>    PRT
<213>    Artificial Sequence

<220>
<223>    clone 2 VL


<400>    63
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Thr Asp
            20                  25                  30

Leu Gly Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Asp Ser Tyr Pro Thr
                85                  90                  95

Thr Phe Gly Gln Gly Thr Lys Val Glu Ile Lys Arg
            100                 105


<210>    64
<211>    324
<212>    DNA
<213>    Artificial Sequence

<220>
<223>    clone 2 VL


<400>    64
gatattcaga tgacacagtc tccttcctct ttgtctgctt ctgtgggcga tagggttaca      60

ataacatgca gggcctctca aggcatctcc actgatttgg gatggtatca gcagaagcct      120

ggaaaggctc ctaaattgtt gatctatgct gcttcttctt tggaatctgg agttccttct      180

agattttctg gatctggatc tggaacagac ttcacattga ccatatcctc cttgcaacct      240

gaagatttcg ctacatatta ctgccaacaa catgattctt atcctactac attcggccag      300

ggaacgaagg tggagattaa gcgt                                             324


<210>    65
<211>    253
<212>    PRT
<213>    Artificial Sequence

<220>
<223>    2D scFv (VH+linker+VL)


<400>    65
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Tyr Met Ser Trp Val Arg Gln Ala Pro Gly Lys Gly Leu Glu Trp Val
        35                  40                  45

Ser Gly Ile Ser Pro Gly Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ala Thr Val Pro Cys Lys Gly Pro Ala Cys Tyr Ser Ala Tyr
            100                 105                 110

Gly Met Asp Val Trp Gly Gln Gly Thr Leu Val Thr Val Ser Ser Gly
        115                 120                 125

Gly Gly Gly Ser Gly Gly Gly Gly Ser Gly Gly Gly Gly Ser Gln Ser
    130                 135                 140

Val Leu Thr Gln Pro Pro Ser Ala Pro Gly Thr Pro Gly Gln Arg Val
145                 150                 155                 160

Thr Ile Ser Cys Ser Gly Ser Ser Ser Asn Ile Gly Asn Asn Ala Val
                165                 170                 175

Asn Trp Tyr Gln Gln Leu Pro Gly Thr Ala Pro Lys Leu Leu Ile Tyr
            180                 185                 190

Ser Asn Ser His Arg Pro Ser Gly Val Pro Asp Arg Phe Ser Gly Ser
        195                 200                 205

Lys Ser Gly Thr Ser Ala Ser Leu Ala Ile Ser Gly Leu Arg Ser Glu
    210                 215                 220

Asp Glu Ala Asp Tyr Tyr Cys Gly Ser Trp Asp Ala Ser Leu Asn Gly
225                 230                 235                 240

Tyr Val Phe Gly Gly Gly Thr Lys Leu Thr Val Leu Gly
                245                 250

<210>   66
<211>   759
<212>   DNA
<213>   Artificial Sequence

<220>
<223>   2D scFv (VH+linker+VL)

<400>   66
gaggtgcagc tgttggagtc tgggggaggc ttggtacagc ctggggggtc cctgagactc      60

tcctgtgcag cctctggatt cacctttagc agttattata tgagctgggt ccgccaggct     120

ccagggaagg ggctggagtg ggtctcaggg atctctcctg gtagtggtag taaatattac     180

gctgattctg taaaaggtcg gttcaccatc tccagagaca attccaagaa cacgctgtat     240

ctgcaaatga acagcctgag agccgaggac acggccgtgt attactgtgc gagagctact     300

gttccgtgta aggggcctgc gtgttattct gcttatggta tggacgtctg gggccagggt     360

acactggtca ccgtgagctc aggtggaggc ggttcaggcg gaggtggatc cggcggtggc 420

ggatcgcagt ctgtgctgac tcagccaccc tcagcgcctg ggacccccgg gcagagggtc 480

accatctctt gtagtggctc ttcatctaat attggcaata atgctgtcaa ctggtaccag 540

cagctcccag gaacggcccc caaactcctc atctattcta atagtcatcg gccaagcggg 600

gtccctgacc gattctctgg ctccaagtct ggcacctcag cctccctggc catcagtggg 660

ctccggtccg aggatgaggc tgattattac tgtggttctt gggatgctag cctgaatggt 720

tatgtcttcg gcggaggcac caagctgacg gtcctaggc 759

<210> 67
<211> 253
<212> PRT
<213> Artificial Sequence

<220>
<223> 4G scFv (VH+linker+VL)

<400> 67
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Leu Ile Ser Ser Gly Asn Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Arg Arg Cys Ser Gly Ala Pro Cys Tyr Ser Ala Tyr
            100                 105                 110

Gly Met Asp Val Trp Gly Gln Gly Thr Leu Val Thr Val Ser Ser Gly
        115                 120                 125

Gly Gly Gly Ser Gly Gly Gly Gly Ser Gly Gly Gly Gly Ser Gln Ser
    130                 135                 140

Val Leu Thr Gln Pro Pro Ser Ala Ser Gly Thr Pro Gly Gln Arg Val
145                 150                 155                 160

Thr Ile Ser Cys Thr Gly Ser Ser Ser Asn Ile Gly Asn Asn Ser Val
                165                 170                 175

Thr Trp Tyr Gln Gln Leu Pro Gly Thr Ala Pro Lys Leu Leu Ile Tyr
            180                 185                 190

Ala Asn Ser Gln Arg Pro Ser Gly Val Pro Asp Arg Phe Ser Gly Ser
        195                 200                 205

Lys Ser Gly Thr Ser Ala Ser Leu Ala Ile Ser Gly Leu Arg Ser Glu
    210                 215                 220

Asp Glu Ala Asp Tyr Tyr Cys Gly Ala Trp Asp Asp Ser Leu Asn Ala
225                 230                 235                 240

Tyr Val Phe Gly Gly Gly Thr Lys Leu Thr Val Leu Gly
                245                 250

<210>   68
<211>   759
<212>   DNA
<213>   Artificial Sequence

<220>
<223>   4G scFv (VH+linker+VL)

<400>   68
gaggtgcagc tgttggagtc tggggggggc ttggtacagc ctggggggtc cctgagactc      60

tcctgtgcag cctctggatt cacctttagc aattattata tgagctgggt ccgccaggct     120

ccagggaagg ggctggagtg ggtctcattg atctcttctg gtaatggtaa taaatattac     180

gctgattctg taaaaggtcg gttcaccatc tccagagaca attccaagaa cacgctgtat     240

ctgcaaatga acagcctgag agccgaggac acggccgtgt attactgtgc gagaggtagt     300

cgtcggtgta gtggtgctcc ttgttattct gcttatggta tggacgtctg gggccagggt     360

acactggtca ccgtgagctc aggtggaggc ggttcaggcg gaggtggatc cggcggtggc     420

ggatcgcagt ctgtgctgac tcagccaccc tcagcgtctg ggacccccgg gcagagggtc     480

accatctctt gtactggctc ttcatctaat attggcaata attctgtcac ctggtaccag     540

cagctcccag gaacggcccc caaactcctc atctatgcta atagtcagcg gccaagcggg     600

gtccctgacc gattctctgg ctccaagtct ggcacctcag cctccctggc catcagtggg     660

ctccggtccg aggatgaggc tgattattac tgtggtgctt gggatgatag cctgaatgct     720

tatgtcttcg gcggaggcac caagctgacg gtcctaggc                            759

<210>   69
<211>   457
<212>   PRT
<213>   Artificial Sequence

<220>
<223>   2D IgG heavy chain

<400>   69
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Tyr Met Ser Trp Val Arg Gln Ala Pro Gly Lys Gly Leu Glu Trp Val
        35                  40                  45

Ser Gly Ile Ser Pro Gly Ser Gly Ser Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Ala Thr Val Pro Cys Lys Gly Pro Ala Cys Tyr Ser Ala Tyr
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Asp Glu Leu
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln

435                 440                 445

Lys Ser Leu Ser Leu Ser Pro Gly Lys
    450                 455

<210>    70
<211>    1371
<212>    DNA
<213>    Artificial Sequence

<220>
<223>    2D IgG heavy chain

<400>    70
gaggtgcagc tgttggagtc tgggggaggc ttggtacagc ctggggggtc cctgagactc      60

tcctgtgcag cctctggatt cacctttagc agttattata tgagctgggt ccgccaggct     120

ccagggaagg ggctggagtg ggtctcaggg atctctcctg gtagtggtag taaatattac     180

gctgattctg taaaaggtcg gttcaccatc tccagagaca attccaagaa cacgctgtat     240

ctgcaaatga acagcctgag agccgaggac acggccgtgt attactgtgc gagagctact     300

gttccgtgta aggggcctgc gtgttattct gcttatggta tggacgtctg gggccagggt     360

acactggtca ccgtgagctc agcctccacc aagggcccat cggtcttccc cctggcaccc     420

tcctccaaga gcacctctgg gggcacagcg gccctgggct gcctggtcaa ggactacttc     480

cccgaaccgg tgacggtgtc gtggaactca ggcgccctga ccagcggcgt gcacaccttc     540

ccggctgtcc tacagtcctc aggactctac tccctcagca gcgtggtgac cgtgccctcc     600

agcagcttgg gcacccagac ctacatctgc aacgtgaatc acaagcccag caacaccaag     660

gtggacaaga aagttgagcc caaatcttgt gacaaaactc acacatgccc accgtgccca     720

gcacctgaac tcctgggggg accgtcagtc ttcctcttcc ccccaaaacc caaggacacc     780

ctcatgatct cccggacccc tgaggtcaca tgcgtggtgg tggacgtgag ccacgaagac     840

cctgaggtca agttcaactg gtacgtggac ggcgtggagg tgcataatgc caagacaaag     900

ccgcgggagg agcagtacaa cagcacgtac cgtgtggtca gcgtcctcac cgtcctgcac     960

caggactggc tgaatggcaa ggagtacaag tgcaaggtct ccaacaaagc cctcccagcc    1020

cccatcgaga aaaccatctc caaagccaaa gggcagcccc gagaaccaca ggtgtatacc    1080

ctgcccccat cccgggatga gctgaccaag aaccaggtca gcctgacctg cctggtcaaa    1140

ggcttctatc ccagcgacat cgccgtggag tgggagagca atgggcagcc ggagaacaac    1200

tacaagacca cgcctcccgt gctggactcc gacggctcct tcttcctcta cagcaagctc    1260

accgtggaca agagcaggtg gcagcagggg aacgtcttct catgctccgt gatgcatgag    1320

gctctgcaca accactacac gcagaagagc ctctccctgt ccccgggtaa a             1371

<210>    71
<211>    216
<212>    PRT
<213>    Artificial Sequence

<220>
<223>    2D IgG light chain

<400>    71
Gln Ser Val Leu Thr Gln Pro Pro Ser Ala Pro Gly Thr Pro Gly Gln
1               5                   10                  15

Arg Val Thr Ile Ser Cys Ser Gly Ser Ser Ser Asn Ile Gly Asn Asn
            20                  25                  30

Ala Val Asn Trp Tyr Gln Gln Leu Pro Gly Thr Ala Pro Lys Leu Leu
        35                  40                  45

Ile Tyr Ser Asn Ser His Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ser Trp Asp Ala Ser Leu
                85                  90                  95

Asn Gly Tyr Val Phe Gly Gly Gly Thr Lys Leu Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Leu Gly Ala Val Thr Val Ala Trp Lys Ala Asp Gly Ser Pro Ile Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Asp Gly Ser Thr Val Glu Lys
        195                 200                 205

Thr Val Ala Pro Thr Glu Cys Ser
    210                 215


<210>    72
<211>    648
<212>    DNA
<213>    Artificial Sequence

<220>
<223>    2D IgG light chain

<400>    72
cagtctgtgc tgactcagcc accctcagcg cctgggaccc ccgggcagag ggtcaccatc      60

tcttgtagtg gctcttcatc taatattggc aataatgctg tcaactggta ccagcagctc     120

ccaggaacgg cccccaaact cctcatctat tctaatagtc atcggccaag cggggtccct     180

gaccgattct ctggctccaa gtctggcacc tcagcctccc tggccatcag tgggctccgg     240

tccgaggatg aggctgatta ttactgtggt tcttgggatg ctagcctgaa tggttatgtc     300

ttcggcggag gcaccaagct gacggtccta ggccagccca aggccaaccc cactgtcact 360

ctgttcccgc cctcctctga ggagctccaa gccaacaagg ccacactagt gtgtctgatc 420

agtgacttct acttgggagc tgtgacagtg gcctggaagg cagatggcag ccccatcaag 480

gcgggagtgg agaccaccaa accctccaaa cagagcaaca acaagtacgc ggccagcagc 540

tacctgagcc tgacgcccga gcagtggaag tcccacagaa gctacagctg ccaggtcacg 600

catgacggga gcaccgtgga gaagacagtg gcccctacag aatgtagc 648

<210> 73
<211> 457
<212> PRT
<213> Artificial Sequence

<220>
<223> 4G IgG heavy chain

<400> 73
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met Ser Trp Val Arg Gln Ala Pro Gly Lys Gly Leu Glu Trp Val
        35                  40                  45

Ser Leu Ile Ser Ser Gly Asn Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Ser Arg Arg Cys Ser Gly Ala Pro Cys Tyr Ser Ala Tyr
            100                 105                 110

Gly Met Asp Val Trp Gly Gln Gly Thr Leu Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Asp Glu Leu
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210>   74
<211>   1371
<212>   DNA
<213>   Artificial Sequence

<220>
<223>   4G IgG heavy chain

<400>   74
gaggtgcagc tgttggagtc tgggggaggc ttggtacagc ctggggggtc cctgagactc      60

tcctgtgcag cctctggatt cacctttagc aattattata tgagctgggt ccgccaggct     120

ccagggaagg ggctggagtg ggtctcattg atctcttctg gtaatggtaa taaatattac     180

gctgattctg taaaaggtcg gttcaccatc tccagagaca attccaagaa cacgctgtat     240

ctgcaaatga acagcctgag agccgaggac acggccgtgt attactgtgc gagaggtagt     300

cgtcggtgta gtggtgctcc ttgttattct gcttatggta tggacgtctg gggccagggt     360

acactggtca ccgtgagctc agcctccacc aagggcccat cggtcttccc cctggcaccc     420

tcctccaaga gcacctctgg gggcacagcg gccctgggct gcctggtcaa ggactacttc     480

cccgaaccgg tgacggtgtc gtggaactca ggcgccctga ccagcggcgt gcacaccttc 540

ccggctgtcc tacagtcctc aggactctac tccctcagca gcgtggtgac cgtgccctcc 600

agcagcttgg gcacccagac ctacatctgc aacgtgaatc acaagcccag caacaccaag 660

gtggacaaga aagttgagcc caaatcttgt gacaaaactc acacatgccc accgtgccca 720

gcacctgaac tcctgggggg accgtcagtc ttcctcttcc ccccaaaacc caaggacacc 780

ctcatgatct cccggacccc tgaggtcaca tgcgtggtgg tggacgtgag ccacgaagac 840

cctgaggtca agttcaactg gtacgtggac ggcgtggagg tgcataatgc caagacaaag 900

ccgcgggagg agcagtacaa cagcacgtac cgtgtggtca gcgtcctcac cgtcctgcac 960

caggactggc tgaatggcaa ggagtacaag tgcaaggtct ccaacaaagc cctcccagcc 1020

cccatcgaga aaaccatctc caaagccaaa gggcagcccc gagaaccaca ggtgtatacc 1080

ctgcccccat cccgggatga gctgaccaag aaccaggtca gcctgacctg cctggtcaaa 1140

ggcttctatc ccagcgacat cgccgtggag tgggagagca atgggcagcc ggagaacaac 1200

tacaagacca cgcctcccgt gctggactcc gacggctcct tcttcctcta cagcaagctc 1260

accgtggaca agagcaggtg gcagcagggg aacgtcttct catgctccgt gatgcatgag 1320

gctctgcaca accactacac gcagaagagc ctctccctgt ccccgggtaa a 1371

<210> 75
<211> 216
<212> PRT
<213> Artificial Sequence

<220>
<223> 4G IgG light chain

<400> 75
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Asn Asn
            20                  25                  30

Ser Val Thr Trp Tyr Gln Gln Leu Pro Gly Thr Ala Pro Lys Leu Leu
        35                  40                  45

Ile Tyr Ala Asn Ser Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Leu
                85                  90                  95

Asn Ala Tyr Val Phe Gly Gly Gly Thr Lys Leu Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Leu Gly Ala Val Thr Val Ala Trp Lys Ala Asp Gly Ser Pro Ile Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Asp Gly Ser Thr Val Glu Lys
        195                 200                 205

Thr Val Ala Pro Thr Glu Cys Ser
    210                 215

<210>    76
<211>    648
<212>    DNA
<213>    Artificial Sequence

<220>
<223>    4G IgG light chain

<400>    76
cagtctgtgc tgactcagcc accctcagcg tctgggaccc ccgggcagag ggtcaccatc      60

tcttgtactg gctcttcatc taatattggc aataattctg tcacctggta ccagcagctc     120

ccaggaacgg cccccaaact cctcatctat gctaatagtc agcggccaag cggggtccct     180

gaccgattct ctggctccaa gtctggcacc tcagcctccc tggccatcag tgggctccgg     240

tccgaggatg aggctgatta ttactgtggt gcttgggatg atagcctgaa tgcttatgtc     300

ttcggcggag gcaccaagct gacggtccta ggccagccca aggccaaccc cactgtcact     360

ctgttcccgc cctcctctga ggagctccaa gccaacaagg ccacactagt gtgtctgatc     420

agtgacttct acttgggagc tgtgacagtg gcctggaagg cagatggcag ccccatcaag     480

gcgggagtgg agaccaccaa accctccaaa cagagcaaca acaagtacgc ggccagcagc     540

tacctgagcc tgacgcccga gcagtggaag tcccacagaa gctacagctg ccaggtcacg     600

catgacggga gcaccgtgga gaagacagtg gcccctacag aatgtagc                  648

<210>    77
<211>    451
<212>    PRT
<213>    Artificial Sequence

<220>
<223>    clone 1 IgG heavy chain

<400>    77
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Trp Met Ser Trp Val Arg Gln Ala Pro Gly Lys Gly Leu Glu Trp Val
           35                  40                  45
   Ser Thr Ile Ser Tyr Gly Gly Gly Gly Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Arg Ser Tyr Gly Ala Ile Pro Pro Asn Thr Ile Asp Tyr Trp Gly
               100                 105                 110
   Gln Gly Thr Leu Val Thr Val Ser Ser Ala Lys Thr Thr Ala Pro Ser
           115                 120                 125
   Val Tyr Pro Leu Ala Pro Val Cys Gly Asp Thr Thr Gly Ser Ser Val
       130                 135                 140
   Thr Leu Gly Cys Leu Val Lys Gly Tyr Phe Pro Glu Pro Val Thr Leu
   145                 150                 155                 160
   Thr Trp Asn Ser Gly Ser Leu Ser Ser Gly Val His Thr Phe Pro Ala
                   165                 170                 175
   Val Leu Gln Ser Asp Leu Tyr Thr Leu Ser Ser Ser Val Thr Val Thr
               180                 185                 190
   Ser Ser Thr Trp Pro Ser Gln Ser Ile Thr Cys Asn Val Ala His Pro
           195                 200                 205
   Ala Ser Ser Thr Lys Val Asp Lys Lys Ile Glu Pro Arg Gly Pro Thr
       210                 215                 220
   Ile Lys Pro Cys Pro Pro Cys Lys Cys Pro Ala Pro Asn Leu Leu Gly
   225                 230                 235                 240
   Gly Pro Ser Val Phe Ile Phe Pro Pro Lys Ile Lys Asp Val Leu Met
                   245                 250                 255
   Ile Ser Leu Ser Pro Ile Val Thr Cys Val Val Val Asp Val Ser Glu
               260                 265                 270
   Asp Asp Pro Asp Val Gln Ile Ser Trp Phe Val Asn Asn Val Glu Val
           275                 280                 285
   His Thr Ala Gln Thr Gln Thr His Arg Glu Asp Tyr Asn Ser Thr Leu
       290                 295                 300
   Arg Val Val Ser Ala Leu Pro Ile Gln His Gln Asp Trp Met Ser Gly
   305                 310                 315                 320
   Lys Glu Phe Lys Cys Lys Val Asn Asn Lys Asp Leu Pro Ala Pro Ile
                   325                 330                 335
   Glu Arg Thr Ile Ser Lys Pro Lys Gly Ser Val Arg Ala Pro Gln Val
               340                 345                 350
   Tyr Val Leu Pro Pro Pro Glu Glu Glu Met Thr Lys Lys Gln Val Thr
           355                 360                 365
   Leu Thr Cys Met Val Thr Asp Phe Met Pro Glu Asp Ile Tyr Val Glu
       370                 375                 380
   Trp Thr Asn Asn Gly Lys Thr Glu Leu Asn Tyr Lys Asn Thr Glu Pro
   385                 390                 395                 400
   Val Leu Asp Ser Asp Gly Ser Tyr Phe Met Tyr Ser Lys Leu Arg Val
   405 410 415
   Glu Lys Lys Asn Trp Val Glu Arg Asn Ser Tyr Ser Cys Ser Val Val
   420 425 430
   His Glu Gly Leu His Asn His His Thr Thr Lys Ser Phe Ser Arg Thr
   435 440 445
   Pro Gly Lys
   450

<210>   78
<211>   1353
<212>   DNA
<213>   Artificial Sequence

<220>
<223>   clone 1 IgG heavy chain

<400>   78
gaagtgcaat tggtggagtc tggcggcgga ttggtgcaac caggaggatc tttgagattg      60

tcttgcgcag cctctggatt cacattctct tattactgga tgagctgggt caggcaggct     120

ccaggaaaag gcttggaatg ggtgtcaacc atttcgtatg gtggaggtgg taaatactac     180

gcagattctg tcaagggcag gttcaccatc tccagggaca actccaagaa cacattgtac     240

ttgcagatga attccttgag agctgaagac acggctgtgt attactgtgt taggtcgtat     300

ggtgcgattc cgcctaatac tattgactac tggggacagg gaacattggt taccgtctct     360

tccgccaaaa caacagcccc atcggtctat ccactggccc ctgtgtgtgg agatacaact     420

ggctcctcgg tgactctagg atgcctggtc aagggttatt tccctgagcc agtgaccttg     480

acctggaact ctggatccct gtccagtggt gtgcacacct tcccagctgt cctgcagtct     540

gacctctaca ccctcagcag ctcagtgact gtaacctcga gcacctggcc cagccagtcc     600

atcacctgca atgtggccca cccggcaagc agcaccaagg tggacaagaa aattgagccc     660

agagggccca caatcaagcc ctgtcctcca tgcaaatgcc cagcacctaa cctcttgggt     720

ggaccatccg tcttcatctt ccctccaaag atcaaggatg tactcatgat ctccctgagc     780

cccatagtca catgtgtggt ggtggatgtg agcgaggatg acccagatgt ccagatcagc     840

tggtttgtga acaacgtgga agtacacaca gctcagacac aaacccatag agaggattac     900

aacagtactc tccgggtggt cagtgccctc cccatccagc accaggactg gatgagtggc     960

aaggagttca aatgcaaggt caacaacaaa gacctcccag cgcccatcga gagaaccatc    1020

tcaaaaccca aagggtcagt aagagctcca caggtatatg tcttgcctcc accagaagaa    1080

gagatgacta agaaacaggt cactctgacc tgcatggtca cagacttcat gcctgaagac    1140

atttacgtgg agtggaccaa caacgggaaa acagagctaa actacaagaa cactgaacca    1200

gtcctggact ctgatggttc ttacttcatg tacagcaagc tgagagtgga aaagaagaac    1260

tgggtggaaa gaaatagcta ctcctgttca gtggtccacg agggtctgca caatcaccac    1320

acgactaaga gcttctcccg gactccgggt aaa                                 1353

<210>    79
<211>    214
<212>    PRT
<213>    Artificial Sequence

<220>
<223>    clone 1 IgG light chain


<400>    79
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Ser Asp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Tyr Ser Thr Pro Ser
                85                  90                  95

Thr Phe Gly Gln Gly Thr Lys Val Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210


<210>    80
<211>    642
<212>    DNA
<213>    Artificial Sequence

<220>
<223>    clone 1 IgG light chain


<400>    80
gatattcaga tgacacagtc tccttcctct ttgtctgctt ctgtgggcga tagggttaca      60

ataacatgca gggcctctca aggcatctcc tctgatttgg catggtatca gcagaagcct      120

ggaaaggctc ctaaattgtt gatctatgct gcttcttctt tgcaatctgg agttccttct 180

agattttctg gatctggatc tggaacagac ttcacattga ccatatcctc cttgcaacct 240

gaagatttcg ctacatatta ctgccaacaa ccttattcta ctccttctac attcggccag 300

ggaacgaagg tggagattaa gcgtgctgat gctgcaccaa ctgtatccat cttcccacca 360

tccagtgagc agttaacatc tggaggtgcc tcagtcgtgt gcttcttgaa caacttctac 420

cccaaagaca tcaatgtcaa gtggaagatt gatggcagtg aacgacaaaa tggcgtcctg 480

aacagttgga ctgatcagga cagcaaagac agcacctaca gcatgagcag caccctcacg 540

ttgaccaagg acgagtatga acgacataac agctatacct gtgaggccac tcacaagaca 600

tcaacttcac ccattgtcaa gagcttcaac aggaatgagt gt 642

<210> 81
<211> 452
<212> PRT
<213> Artificial Sequence

<220>
<223> clone 2 IgG heavy chain

<400> 81
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Trp Met Ser Trp Val Arg Gln Ala Pro Gly Lys Gly Leu Glu Trp Val
        35                  40                  45

Ser Val Ile Ser Tyr Asp Gly Gly Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Trp Ala Asn Asp His Asn Ser Asp Leu Asn Phe Asp Tyr Trp
            100                 105                 110

Gly Gln Gly Thr Leu Val Thr Val Ser Ser Ala Lys Thr Thr Ala Pro
        115                 120                 125

Ser Val Tyr Pro Leu Ala Pro Val Cys Gly Asp Thr Thr Gly Ser Ser
    130                 135                 140

Val Thr Leu Gly Cys Leu Val Lys Gly Tyr Phe Pro Glu Pro Val Thr
145                 150                 155                 160

Leu Thr Trp Asn Ser Gly Ser Leu Ser Ser Gly Val His Thr Phe Pro
                165                 170                 175

Ala Val Leu Gln Ser Asp Leu Tyr Thr Leu Ser Ser Ser Val Thr Val
            180                 185                 190

Thr Ser Ser Thr Trp Pro Ser Gln Ser Ile Thr Cys Asn Val Ala His
        195                 200                 205

Pro Ala Ser Ser Thr Lys Val Asp Lys Lys Ile Glu Pro Arg Gly Pro
    210                 215                 220

Thr Ile Lys Pro Cys Pro Pro Cys Lys Cys Pro Ala Pro Asn Leu Leu
225                 230                 235                 240

Gly Gly Pro Ser Val Phe Ile Phe Pro Pro Lys Ile Lys Asp Val Leu
                245                 250                 255

Met Ile Ser Leu Ser Pro Ile Val Thr Cys Val Val Val Asp Val Ser
            260                 265                 270

Glu Asp Asp Pro Asp Val Gln Ile Ser Trp Phe Val Asn Asn Val Glu
        275                 280                 285

Val His Thr Ala Gln Thr Gln Thr His Arg Glu Asp Tyr Asn Ser Thr
    290                 295                 300

Leu Arg Val Val Ser Ala Leu Pro Ile Gln His Gln Asp Trp Met Ser
305                 310                 315                 320

Gly Lys Glu Phe Lys Cys Lys Val Asn Asn Lys Asp Leu Pro Ala Pro
                325                 330                 335

Ile Glu Arg Thr Ile Ser Lys Pro Lys Gly Ser Val Arg Ala Pro Gln
            340                 345                 350

Val Tyr Val Leu Pro Pro Pro Glu Glu Glu Met Thr Lys Lys Gln Val
        355                 360                 365

Thr Leu Thr Cys Met Val Thr Asp Phe Met Pro Glu Asp Ile Tyr Val
    370                 375                 380

Glu Trp Thr Asn Asn Gly Lys Thr Glu Leu Asn Tyr Lys Asn Thr Glu
385                 390                 395                 400

Pro Val Leu Asp Ser Asp Gly Ser Tyr Phe Met Tyr Ser Lys Leu Arg
                405                 410                 415

Val Glu Lys Lys Asn Trp Val Glu Arg Asn Ser Tyr Ser Cys Ser Val
            420                 425                 430

Val His Glu Gly Leu His Asn His His Thr Thr Lys Ser Phe Ser Arg
        435                 440                 445

Thr Pro Gly Lys
    450

<210> 82
<211> 1356
<212> DNA
<213> Artificial Sequence

<220>
<223> clone 2 IgG heavy chain

<400> 82
gaagtgcaat tggtggagtc tggcggcgga ttggtgcaac caggaggatc tttgagattg 60

tcttgcgcag cctctggatt cacattctct tcttactgga tgagctgggt caggcaggct 120

ccaggaaaag gcttggaatg ggtgtcagtc atttcgtatg atggaggtaa taaatactac 180

gcagattctg tcaagggcag gttcaccatc tccagggaca actccaagaa cacattgtac 240

ttgcagatga attccttgag agctgaagac acggctgtgt attactgtgc taagtgggct 300

aatgatcata attctgatct taattttgac tactggggac agggaacatt ggttaccgtc 360

tcttccgcca aaacaacagc cccatcggtc tatccactgg cccctgtgtg tggagataca 420

actggctcct cggtgactct aggatgcctg gtcaagggtt atttccctga gccagtgacc 480

ttgacctgga actctggatc cctgtccagt ggtgtgcaca ccttcccagc tgtcctgcag 540

tctgacctct acaccctcag cagctcagtg actgtaacct cgagcacctg gcccagccag 600

tccatcacct gcaatgtggc ccacccggca agcagcacca aggtggacaa gaaaattgag 660

cccagagggc ccacaatcaa gccctgtcct ccatgcaaat gcccagcacc taacctcttg 720

ggtggaccat ccgtcttcat cttccctcca aagatcaagg atgtactcat gatctccctg 780

agccccatag tcacatgtgt ggtggtggat gtgagcgagg atgacccaga tgtccagatc 840

agctggtttg tgaacaacgt ggaagtacac acagctcaga cacaaaccca tagagaggat 900

tacaacagta ctctccgggt ggtcagtgcc ctccccatcc agcaccagga ctggatgagt 960

ggcaaggagt tcaaatgcaa ggtcaacaac aaagacctcc cagcgcccat cgagagaacc 1020

atctcaaaac ccaaagggtc agtaagagct ccacaggtat atgtcttgcc tccaccagaa 1080

gaagagatga ctaagaaaca ggtcactctg acctgcatgg tcacagactt catgcctgaa 1140

gacatttacg tggagtggac caacaacggg aaaacagagc taaactacaa gaacactgaa 1200

ccagtcctgg actctgatgg ttcttacttc atgtacagca agctgagagt ggaaaagaag 1260

aactgggtgg aaagaaatag ctactcctgt tcagtggtcc acgagggtct gcacaatcac 1320

cacacgacta agagcttctc ccggactccg ggtaaa 1356

<210> 83
<211> 214
<212> PRT
<213> Artificial Sequence

<220>
<223> clone 2 IgG light chain

<400> 83
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Ser Thr Asp
            20                  25                  30

Leu Gly Trp Tyr Gln Gln Lys Pro Gly Lys Ala Pro Lys Leu Leu Ile
        35                  40                  45

Tyr Ala Ala Ser Ser Leu Glu Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Asp Ser Tyr Pro Thr
                85                  90                  95

Thr Phe Gly Gln Gly Thr Lys Val Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210

<210>    84
<211>    642
<212>    DNA
<213>    Artificial Sequence

<220>
<223>    clone 2 IgG light chain

<400>    84
gatattcaga tgacacagtc tccttcctct ttgtctgctt ctgtgggcga tagggttaca      60

ataacatgca gggcctctca aggcatctcc actgatttgg gatggtatca gcagaagcct     120

ggaaaggctc ctaaattgtt gatctatgct gcttcttctt tggaatctgg agttccttct     180

agattttctg gatctggatc tggaacagac ttcacattga ccatatcctc cttgcaacct     240

gaagatttcg ctacatatta ctgccaacaa catgattctt atcctactac attcggccag     300

ggaacgaagg tggagattaa gcgtgctgat gctgcaccaa ctgtatccat cttcccacca     360

tccagtgagc agttaacatc tggaggtgcc tcagtcgtgt gcttcttgaa caacttctac     420

cccaaagaca tcaatgtcaa gtggaagatt gatggcagtg aacgacaaaa tggcgtcctg     480

aacagttgga ctgatcagga cagcaaagac agcacctaca gcatgagcag caccctcacg     540

ttgaccaagg acgagtatga acgacataac agctatacct gtgaggccac tcacaagaca     600

tcaacttcac ccattgtcaa gagcttcaac aggaatgagt gt                        642

<210>    85
<211>    246
<212>    PRT
<213>    Artificial Sequence

<220>
<223>    PICP alpha 1

<400>    85
Asp Asp Ala Asn Val Val Arg Asp Arg Asp Leu Glu Val Asp Thr Thr
1               5                   10                  15

Leu Lys Ser Leu Ser Gln Gln Ile Glu Asn Ile Arg Ser Pro Glu Gly
            20                  25                  30

Ser Arg Lys Asn Pro Ala Arg Thr Cys Arg Asp Leu Lys Met Cys His
        35                  40                  45

Ser Asp Trp Lys Ser Gly Glu Tyr Trp Ile Asp Pro Asn Gln Gly Cys
    50                  55                  60

Asn Leu Asp Ala Ile Lys Val Phe Cys Asn Met Glu Thr Gly Glu Thr
65                  70                  75                  80

Cys Val Tyr Pro Thr Gln Pro Ser Val Ala Gln Lys Asn Trp Tyr Ile
                85                  90                  95

Ser Lys Asn Pro Lys Asp Lys Arg His Val Trp Phe Gly Glu Ser Met
            100                 105                 110

Thr Asp Gly Phe Gln Phe Glu Tyr Gly Gly Gln Gly Ser Asp Pro Ala
        115                 120                 125

Asp Val Ala Ile Gln Leu Thr Phe Leu Arg Leu Met Ser Thr Glu Ala
    130                 135                 140

Ser Gln Asn Ile Thr Tyr His Cys Lys Asn Ser Val Ala Tyr Met Asp
145                 150                 155                 160

Gln Gln Thr Gly Asn Leu Lys Lys Ala Leu Leu Leu Gln Gly Ser Asn
                165                 170                 175

Glu Ile Glu Ile Arg Ala Glu Gly Asn Ser Arg Phe Thr Tyr Ser Val
            180                 185                 190

Thr Val Asp Gly Cys Thr Ser His Thr Gly Ala Trp Gly Lys Thr Val
        195                 200                 205

Ile Glu Tyr Lys Thr Thr Lys Thr Ser Arg Leu Pro Ile Ile Asp Val
    210                 215                 220

Ala Pro Leu Asp Val Gly Ala Pro Asp Gln Glu Phe Gly Phe Asp Val
225                 230                 235                 240

Gly Pro Val Cys Phe Leu
                245

<210> 86
<211> 738
<212> DNA
<213> Artificial Sequence

<220>
<223> PICP alpha 1

<400> 86
gatgatgcca atgtggttcg tgaccgtgac ctcgaggtgg acaccaccct caagagcctg 60

agccagcaga tcgagaacat ccggagccca gagggcagcc gcaagaaccc cgcccgcacc 120

tgccgtgacc tcaagatgtg ccactctgac tggaagagtg gagagtactg gattgacccc 180

aaccaaggct gcaacctgga tgccatcaaa gtcttctgca acatggagac tggtgagacc 240

tgcgtgtacc ccactcagcc cagtgtggcc cagaagaact ggtacatcag caagaacccc 300

aaggacaaga ggcatgtctg gttcggcgag agcatgaccg atggattcca gttcgagtat 360

ggcggccagg gctccgaccc tgccgatgtg gccatccagc tgaccttcct gcgcctgatg 420

tccaccgagg cctcccagaa catcacctac cactgcaaga acagcgtggc ctacatggac 480

cagcagactg gcaacctcaa gaaggccctg ctcctccagg gctccaacga gatcgagatc 540

cgcgccgagg gcaacagccg cttcacctac agcgtcactg tcgatggctg cacgagtcac 600

accggagcct ggggcaagac agtgattgaa tacaaaacca ccaagacctc ccgcctgccc 660

atcatcgatg tggccccctt ggacgttggt gccccagacc aggaattcgg cttcgacgtt 720

ggccctgtct gcttcctg 738

<210> 87
<211> 247
<212> PRT
<213> Artificial Sequence

<220>
<223> PICP alpha 2

<400> 87
Asp Gln Pro Arg Ser Ala Pro Ser Leu Arg Pro Lys Asp Tyr Glu Val
1               5                   10                  15

Asp Ala Thr Leu Lys Ser Leu Asn Asn Gln Ile Glu Thr Leu Leu Thr
            20                  25                  30

Pro Glu Gly Ser Arg Lys Asn Pro Ala Arg Thr Cys Arg Asp Leu Arg
        35                  40                  45

Leu Ser His Pro Glu Trp Ser Ser Gly Tyr Tyr Trp Ile Asp Pro Asn
    50                  55                  60

Gln Gly Cys Thr Met Asp Ala Ile Lys Val Tyr Cys Asp Phe Ser Thr
65                  70                  75                  80

Gly Glu Thr Cys Ile Arg Ala Gln Pro Glu Asn Ile Pro Ala Lys Asn
                85                  90                  95

Trp Tyr Arg Ser Ser Lys Asp Lys Lys His Val Trp Leu Gly Glu Thr
            100                 105                 110

Ile Asn Ala Gly Ser Gln Phe Glu Tyr Asn Val Glu Gly Val Thr Ser
        115                 120                 125

Lys Glu Met Ala Thr Gln Leu Ala Phe Met Arg Leu Leu Ala Asn Tyr
    130                 135                 140

Ala Ser Gln Asn Ile Thr Tyr His Cys Lys Asn Ser Ile Ala Tyr Met
145                 150                 155                 160

Asp Glu Glu Thr Gly Asn Leu Lys Lys Ala Val Ile Leu Gln Gly Ser
                165                 170                 175

Asn Asp Val Glu Leu Val Ala Glu Gly Asn Ser Arg Phe Thr Tyr Thr
            180                 185                 190

Val Leu Val Asp Gly Cys Ser Lys Lys Thr Asn Glu Trp Gly Lys Thr
        195                 200                 205

Ile Ile Glu Tyr Lys Thr Asn Lys Pro Ser Arg Leu Pro Phe Leu Asp
    210                 215                 220

Ile Ala Pro Leu Asp Ile Gly Gly Ala Asp Gln Glu Phe Phe Val Asp
225                 230                 235                 240

Ile Gly Pro Val Cys Phe Lys
                245

<210>   88
<211>   741
<212>   DNA
<213>   Artificial Sequence

<220>
<223>   PICP alpha 2

<400>   88
gaccagcctc gctcagcacc ttctctcaga cccaaggact atgaagttga tgctactctg      60

aagtctctca acaaccagat tgagaccctt cttactcctg aaggctctag aaagaaccca     120

gctcgcacat gccgtgactt gagactcagc cacccagagt ggagcagtgg ttactactgg     180

attgacccta accaaggatg cactatggat gctatcaaag tatactgtga tttctctact     240

ggcgaaacct gtatccgtgc ccaacctgaa aacatcccag ccaagaactg gtataggagc     300

tccaaggaca agaaacacgt ctggctagga gaaactatca atgctggcag ccagtttgaa     360

tataatgtag aaggagtgac ttccaaggaa atggctaccc aacttgcctt catgcgcctg     420

ctggccaact atgcctctca gaacatcacc taccactgca agaacagcat tgcatacatg     480

gatgaggaga ctggcaacct gaaaaaggct gtcattctac agggctctaa tgatgttgaa     540

cttgttgctg agggcaacag caggttcact tacactgttc ttgtagatgg ctgctctaaa     600

aagacaaatg aatggggaaa gacaatcatt gaatacaaaa caaataagcc atcacgcctg     660

cccttccttg atattgcacc tttggacatc ggtggtgctg accaggaatt ctttgtggac     720

attggcccag tctgtttcaa a                                               741

<210>   89
<211>   243
<212>   PRT
<213>   Artificial Sequence

<220>
<223>   PIIICP alpha 1

<400>   89
Asp Glu Pro Met Asp Phe Lys Ile Asn Thr Asp Glu Ile Met Thr Ser
1               5                   10                  15

Leu Lys Ser Val Asn Gly Gln Ile Glu Ser Leu Ile Ser Pro Asp Gly
            20                  25                  30

Ser Arg Lys Asn Pro Ala Arg Asn Cys Arg Asp Leu Lys Phe Cys His
        35                  40                  45

Pro Glu Leu Lys Ser Gly Glu Tyr Trp Val Asp Pro Asn Gln Gly Cys
    50                  55                  60

Lys Leu Asp Ala Ile Lys Val Phe Cys Asn Met Glu Thr Glu Thr Cys
65                  70                  75                  80

Ile Ser Ala Asn Pro Leu Asn Val Pro Arg Lys His Trp Trp Thr Asp
                85                  90                  95

Ser Ser Ala Glu Lys Lys His Val Trp Phe Gly Glu Ser Met Asp Gly
            100                 105                 110

Gly Phe Gln Phe Ser Tyr Gly Asn Pro Glu Leu Pro Glu Asp Val Leu
        115                 120                 125

Asp Val Gln Leu Ala Phe Leu Arg Leu Ser Ser Arg Ala Ser Gln Asn
    130                 135                 140

Ile Thr Tyr His Cys Lys Asn Ser Ile Ala Tyr Met Asp Gln Ala Ser
145                 150                 155                 160

Gly Asn Val Lys Lys Ala Leu Lys Leu Met Gly Ser Asn Glu Gly Glu
                165                 170                 175

Phe Lys Ala Glu Gly Asn Ser Lys Phe Thr Tyr Thr Val Leu Glu Asp
            180                 185                 190

Gly Cys Thr Lys His Thr Gly Glu Trp Ser Lys Thr Val Phe Glu Tyr
        195                 200                 205

Arg Thr Arg Lys Ala Val Arg Leu Pro Ile Val Asp Ile Ala Pro Tyr
    210                 215                 220

Asp Ile Gly Gly Pro Asp Gln Glu Phe Gly Val Asp Val Gly Pro Val
225                 230                 235                 240

Cys Phe Leu


<210>   90
<211>   729
<212>   DNA
<213>   Artificial Sequence

<220>
<223>   PIIICP alpha 1

<400>   90
gatgaaccaa tggatttcaa aatcaacacc gatgagatta tgacttcact caagtctgtt      60

aatggacaaa tagaaagcct cattagtcct gatggttctc gtaaaaaccc cgctagaaac     120

tgcagagacc tgaaattctg ccatcctgaa ctcaagagtg gagaatactg ggttgaccct     180

aaccaaggat gcaaattgga tgctatcaag gtattctgta atatggaaac tgaaacatgc     240

ataagtgcca atcctttgaa tgttccacgg aaacactggt ggacagattc tagtgctgag     300

aagaaacacg tttggtttgg agagtccatg gatggtggtt ttcagtttag ctacggcaat     360

cctgaacttc ctgaagatgt ccttgatgtg cagctggcat tccttcgact ttccagccga     420

gcttcccaga acatcacata tcactgcaaa aatagcattg catacatgga tcaggccagt     480

ggaaatgtaa agaaggccct gaagctgatg gggtcaaatg aaggtgaatt caaggctgaa     540

ggaaatagca aattcaccta cacagttctg gaggatggtt gcacgaaaca cactggggaa     600

tggagcaaaa cagtctttga atatcgaaca cgcaaggctg tgagactacc tattgtagat     660

attgcaccct atgacattgg tggtcctgat caagaatttg gtgtggacgt tggccctgtt     720

tgcttttta                                                          729

<210>   91
<211>   62
<212>   DNA
<213>   Artificial Sequence

<220>
<223>   PICP alpha 1 forward primer

<400>   91
aaaaggcgcg cccatcacca tcaccatcac catcacggcg ccgatgatgc caatgtggtt      60

cg                                                                     62

<210>   92
<211>   31
<212>   DNA
<213>   Artificial Sequence

<220>
<223>   PICP alpha 1 reverse primer

<400>   92
ttgggccctt actacaggaa gcagacaggg c                                     31

<210>   93
<211>   64
<212>   DNA
<213>   Artificial Sequence

<220>
<223>   PICP alpha 2 N-term forward primer

<400>   93
aaaaggcgcg ccgaacagaa actgatctct gaagaagacc tgggcgccga ccagcctcgc      60

tcag                                                                   64

<210>   94
<211>   37
<212>   DNA
<213>   Artificial Sequence

<220>
<223>   PICP alpha 2 N-term reverse primer

<400>   94
ggatgttttc aggttgggca cggatacagg tttcgcc                                37

<210>   95
<211>   19
<212>   DNA
<213>   Artificial Sequence

<220>
<223>   PICP alpha 2 C-term forward primer

<400>    95
gcccaacctg aaaacatcc                                                    19


<210>    96
<211>    36
<212>    DNA
<213>    Artificial Sequence

<220>
<223>    PICP alpha 2 C-term reverse primer


<400>    96
ttgggcccct attatttgaa acagactggg ccaatg                                 36


<210>    97
<211>    66
<212>    DNA
<213>    Artificial Sequence

<220>
<223>    PIICP forward primer


<400>    97
aaaaggcgcg cccatcacca tcaccatcac catcacggcg ccgatgaacc aatggatttc        60

aaaatc                                                                  66


<210>    98
<211>    35
<212>    DNA
<213>    Artificial Sequence

<220>
<223>    PIICP reverse primer


<400>    98
ttgggcccct attataaaaa gcaaacaggg ccaac                                  35


<210>    99
<211>    36
<212>    DNA
<213>    Artificial Sequence

<220>
<223>    scFv VH forward primer


<400>    99
agagagtgta cactcccagg cggccgaggt gcagcg                                 36


<210>    100
<211>    42
<212>    DNA
<213>    Artificial Sequence

<220>
<223>    scFv VH reverse primer

<400>    100
cgccgctggg cccttggtgg aggctgagct cacggtgacc ag                          42


<210>    101
<211>    89
<212>    DNA
<213>    Artificial Sequence

<220>
<223>    scFv VL forward primer


<400>    101
aagcggccgc caccatggga tggagctgta tcatcctctt cttggtagca acagctacag       60

gtgtacactc ccagtctgtg ctgactcag                                         89


<210>    102
<211>    30
<212>    DNA
<213>    Artificial Sequence

<220>
<223>    scFv VL reverse primer


<400>    102
cgccgccgta cgtaggaccg tcagcttggt                                        30


<210>    103
<211>    34
<212>    DNA
<213>    Artificial Sequence

<220>
<223>    Fab VH forward primer


<400>    103
aatgtacact ccgaagtgca attggtggag tctg                                   34


<210>    104
<211>    40
<212>    DNA
<213>    Artificial Sequence

<220>
<223>    Fab VH reverse primer


<400>    104
gaccgatggg gctgttgttt tggcggaaga gacggtaacc                             40


<210>    105
<211>    24
<212>    DNA
<213>    Artificial Sequence

<220>
<223>    mIgG2a CH1-CH3 forward primer


<400>    105

gccaaaacaa cagccccatc ggtc 24

<210> 106
<211> 37
<212> DNA
<213> Artificial Sequence

<220>
<223> mIgG2a CH1-CH3 reverse primer

<400> 106
atatccaagc ttctactatt tacccggagt ccgggag 37

<210> 107
<211> 45
<212> DNA
<213> Artificial Sequence

<220>
<223> Fab VL forward primer

<400> 107
ctcggtcata atgtccagag gagatattca gatgacacag tctcc 45

<210> 108
<211> 24
<212> DNA
<213> Artificial Sequence

<220>
<223> Fab VL reverse primer

<400> 108
ttcgtacgct taatctccac cttc 24

<210> 109
<211> 41
<212> DNA
<213> Artificial Sequence

<220>
<223> mIgG2a kappa chain forward primer

<400> 109
cttttacatt cggccaggga acgaaggtgg agattaagcg t 41

<210> 110
<211> 35
<212> DNA
<213> Artificial Sequence

<220>
<223> mIgG2a kappa chain reverse primer

<400> 110
atccaagctt ctactaacac tcattcctgt tgaag 35

<210>	111
<211>	285
<212>	PRT
<213>	Artificial Sequence

<220>
<223>	PICP alpha 1 protein with murine Ig k-chain V-J2-C signal peptide
	and His tag on the N-terminus


<400>	111
Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Arg Ala His His His
            20                  25                  30

His His His His His Gly Ala Asp Asp Ala Asn Val Val Arg Asp Arg
        35                  40                  45

Asp Leu Glu Val Asp Thr Thr Leu Lys Ser Leu Ser Gln Gln Ile Glu
    50                  55                  60

Asn Ile Arg Ser Pro Glu Gly Ser Arg Lys Asn Pro Ala Arg Thr Cys
65                  70                  75                  80

Arg Asp Leu Lys Met Cys His Ser Asp Trp Lys Ser Gly Glu Tyr Trp
                85                  90                  95

Ile Asp Pro Asn Gln Gly Cys Asn Leu Asp Ala Ile Lys Val Phe Cys
            100                 105                 110

Asn Met Glu Thr Gly Glu Thr Cys Val Tyr Pro Thr Gln Pro Ser Val
        115                 120                 125

Ala Gln Lys Asn Trp Tyr Ile Ser Lys Asn Pro Lys Asp Lys Arg His
    130                 135                 140

Val Trp Phe Gly Glu Ser Met Thr Asp Gly Phe Gln Phe Glu Tyr Gly
145                 150                 155                 160

Gly Gln Gly Ser Asp Pro Ala Asp Val Ala Ile Gln Leu Thr Phe Leu
                165                 170                 175

Arg Leu Met Ser Thr Glu Ala Ser Gln Asn Ile Thr Tyr His Cys Lys
            180                 185                 190

Asn Ser Val Ala Tyr Met Asp Gln Gln Thr Gly Asn Leu Lys Lys Ala
        195                 200                 205

Leu Leu Leu Gln Gly Ser Asn Glu Ile Glu Ile Arg Ala Glu Gly Asn
    210                 215                 220

Ser Arg Phe Thr Tyr Ser Val Thr Val Asp Gly Cys Thr Ser His Thr
225                 230                 235                 240

Gly Ala Trp Gly Lys Thr Val Ile Glu Tyr Lys Thr Thr Lys Thr Ser
                245                 250                 255

Arg Leu Pro Ile Ile Asp Val Ala Pro Leu Asp Val Gly Ala Pro Asp
            260                 265                 270

Gln Glu Phe Gly Phe Asp Val Gly Pro Val Cys Phe Leu
        275                 280                 285


<210>	112
<211>	288

<212>   PRT
<213>   Artificial Sequence

<220>
<223>   PICP alpha 2 protein with murine Ig k-chain V-J2-C signal peptide
        and C-myc tag on the N-terminus

<400>   112
Met Glu Thr Asp Thr Leu Leu Leu Trp Val Leu Leu Leu Trp Val Pro
1               5                   10                  15

Gly Ser Thr Gly Asp Ala Ala Gln Pro Ala Arg Arg Ala Glu Gln Lys
            20                  25                  30

Leu Ile Ser Glu Glu Asp Leu Gly Ala Asp Gln Pro Arg Ser Ala Pro
        35                  40                  45

Ser Leu Arg Pro Lys Asp Tyr Glu Val Asp Ala Thr Leu Lys Ser Leu
    50                  55                  60

Asn Asn Gln Ile Glu Thr Leu Leu Thr Pro Glu Gly Ser Arg Lys Asn
65                  70                  75                  80

Pro Ala Arg Thr Cys Arg Asp Leu Arg Leu Ser His Pro Glu Trp Ser
                85                  90                  95

Ser Gly Tyr Tyr Trp Ile Asp Pro Asn Gln Gly Cys Thr Met Asp Ala
            100                 105                 110

Ile Lys Val Tyr Cys Asp Phe Ser Thr Gly Glu Thr Cys Ile Arg Ala
        115                 120                 125

Gln Pro Glu Asn Ile Pro Ala Lys Asn Trp Tyr Arg Ser Ser Lys Asp
    130                 135                 140

Lys Lys His Val Trp Leu Gly Glu Thr Ile Asn Ala Gly Ser Gln Phe
145                 150                 155                 160

Glu Tyr Asn Val Glu Gly Val Thr Ser Lys Glu Met Ala Thr Gln Leu
                165                 170                 175

Ala Phe Met Arg Leu Leu Ala Asn Tyr Ala Ser Gln Asn Ile Thr Tyr
            180                 185                 190

His Cys Lys Asn Ser Ile Ala Tyr Met Asp Glu Glu Thr Gly Asn Leu
        195                 200                 205

Lys Lys Ala Val Ile Leu Gln Gly Ser Asn Asp Val Glu Leu Val Ala
    210                 215                 220

Glu Gly Asn Ser Arg Phe Thr Tyr Thr Val Leu Val Asp Gly Cys Ser
225                 230                 235                 240

Lys Lys Thr Asn Glu Trp Gly Lys Thr Ile Ile Glu Tyr Lys Thr Asn
                245                 250                 255

Lys Pro Ser Arg Leu Pro Phe Leu Asp Ile Ala Pro Leu Asp Ile Gly
            260                 265                 270

Gly Ala Asp Gln Glu Phe Phe Val Asp Ile Gly Pro Val Cys Phe Lys
        275                 280                 285